(19) Europäisches Patentamt
European Patent Office
Office européen des brevets

(11) **EP 2 242 598 B1**

(12) **EUROPÄISCHE PATENTSCHRIFT**

(45) Veröffentlichungstag und Bekanntmachung des Hinweises auf die Patenterteilung:
**28.12.2011 Patentblatt 2011/52**

(21) Anmeldenummer: **09705452.2**

(22) Anmeldetag: **28.01.2009**

(51) Int Cl.:
*B21H 7/00* (2006.01)          *A61B 17/30* (2006.01)
*B25B 9/02* (2006.01)          *A61C 13/28* (2006.01)
*A61F 2/30* (2006.01)

(86) Internationale Anmeldenummer:
**PCT/CH2009/000033**

(87) Internationale Veröffentlichungsnummer:
**WO 2009/094794 (06.08.2009 Gazette 2009/32)**

(54) **EINZYLINDER-SCHUBWALZVERFAHREN, VORRICHTUNG HIERZU UND DAMIT HERGESTELLTE ERZEUGNISSE**

ONE-CYLINDER THRUST ROLL METHOD, DEVICE THEREFOR AND PRODUCTS MANUFACTURED THEREWITH

PROCÉDÉ DE LAMINAGE PAR POUSSÉE À UN CYLINDRE, DISPOSITIF ASSOCIÉ ET PRODUITS AINSI FABRIQUÉS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **31.01.2008 CH 141082008**

(43) Veröffentlichungstag der Anmeldung:
**27.10.2010 Patentblatt 2010/43**

(73) Patentinhaber: **DUMONT SWITZERLAND AG**
**6302 Zug (CH)**

(72) Erfinder: **WALLISER, Anton**
**CH-4051 Basel (CH)**

(74) Vertreter: **Kaufmann, Michael Charles**
**Bohest AG**
**Holbeinstrasse 36-38**
**Postfach 160**
**CH-4003 Basel (CH)**

(56) Entgegenhaltungen:
**EP-A- 0 226 962          EP-A- 1 080 657**
**DE-A1- 1 527 680          DE-U1- 20 012 425**
**DE-U1- 29 906 571          US-A- 1 898 061**
**US-A- 2 666 988          US-A- 2 827 689**
**US-A- 3 457 759          US-A- 5 001 918**

EP 2 242 598 B1

## Beschreibung

### Verweis auf frühere Anmeldungen

Diese Anmeldung beansprucht die Priorität der Schweizer Patentanmeldung Nr. 141/08.

### Gebiet der Erfindung

Die vorliegende Erfindung betrifft die Herstellung von Präzisionsinstrumenten wie etwa Pinzetten.

Diese Instrumente weisen zwei Schenkel auf, die an dem einen Ende miteinander verbunden sind, etwa mittels Punktschweissen. Die anderen beiden freien Enden der beiden Schenkel stehen etwas auseinandergespreizt voneinander ab und können federnd zusammengedrückt werden. Die freien Enden der Schenkel können etwa im Fall einer Pinzette zu feinen Spitzen ausgeformt sein, die genau aufeinander passen müssen, damit die Pinzette ihre Funktion erfüllt. Da die beiden Schenkel erst nach der Herstellung ihrer freien Spitzen am anderen Ende miteinander verbunden werden, müssen die Pinzetten in der Regel anschliessend noch von Hand nachbearbeitet werden, damit die Spitzen aufeinander passen.

Endlose metallischer Formteile wie etwa Schienen, Profile oder Drähte werden andererseits, wie bekannt, mittels Zweizylinderwalzenwerken hergestellt. Diese üben eine symmetrisch quetschende Wirkung auf das Metall aus. Die Kristallgitterachsen der neu geschichteten Struktur werden von der Mitte in Walzrichtung sternförmig nach beiden Seiten ausgerichtet; es bildet sich vor den Walzen ein sogenannter "Walzlöffel" aus. Das Walzgut wächst bei diesem Walzverfahren somit in die Länge wie auch in die Breite. Das Breitenwachstum des Walzgutes kann nicht dadurch verhindert werden, dass in den Walzzylindern seitliche Begrenzungen oder Öffnungen vorgesehen werden, da die seitwärts gerichteten Kräfte im sich ausbreitenden Material eine Sprengwirkung mit Gratbildung erzeugen würden. Dies würde zum Bruch der Walzzylinder oder zum Bruch oder Blockieren der ganzen Maschine führen. Um die Ausdehnung des Walzgutes in die Breite zu verhindern muss bei Zweizylinderwalzwerken eine nachgeschaltete Zugvorrichtung vorgesehen werden, die einen derartig grossen Zug auf das Walzgut ausübt, dass es sich beim Walzvorgang nur noch unwesentlich in die Breite ausdehnen kann.

### Stand der Technik

In der EP 1 275 472 A wird in Paragraph 5 erwähnt, dass sich komplexe Konturen durch Walzen mit einrolligen mechanischen Werkzeugen herstellen lassen, wobei der Walzvorgang an einem Wirkpunkt zwischen Rolle und Werkzeugoberfläche stattfindet.

In der WO 01/13756 A wird eine Pinzette aus Leichtmetall offenbart, die aus einem Stück besteht und die keine Schweissstellen aufweist. Sie wird durch Zerlegen eines Leichtmetall-Strangprofils in eine Mehrzahl solcher Pinzetten, ohne Walzen, hergestellt.

Das Walzen von Metallstreifen ist auch aus der US-A-5 001 918, der US-A-1 898 061, der US-A-3 457 759 und der DE-A-1 527 680 bekannt.

Die vorliegende Erfindung setzt sich erstens zum Ziel, ein Zwischenprodukt in Form eines Metallstreifens mit neuen Materialeigenschaften bereitzustellen, das zur Herstellung von Instrumenten der eingangs erwähnten Art geeignet ist. Sie setzt sich auch zum Ziel, ein Herstellungsverfahren und die zugehörige Vorrichtung zum Erhalt dieses Metallstreifens bereitzustellen, wobei dieses Verfahren in dem Metallstreifen insbesondere die federnden Teile der Schenkel von Instrumenten der eingangs erwähnten Art ergibt. Sie setzt sich auch zum Ziel, ein Herstellungsverfahren für andere metallische Gegenstände bereitzustellen, die einen federnden Bereich enthalten oder komplexe Profilformen aufweisen.

### Zusammenfassung der Erfindung

Die Aufgabe wird erfindungsgemäss nach Anspruch 1 gelöst durch einen einstückigen Metallstreifen ohne Schweissnähte und aus einer polykristallinen Stahllegierung, umfassend mindestens einen Bereich, in dem die Kristallite vergleichsweise stärker anisotrop orientiert sind, und mindestens einen Bereich, in dem die Kristallite vergleichsweise weniger stark anisotrop orientiert sind; und wobei mit CuK$\alpha$-Strahlung an zwei beliebigen Stellen des Streifens gemessene $\theta$-$2\theta$-Röntgendiffraktogramme keine statistisch signifikanten Unterschiede hinsichtlich Lage und Form der jeweils entsprechenden Piken zeigen. Die vergleichsweise stärker anisotrope Kristallitorientierung des einen Bereichs ist im Vergleich zu der vergleichsweise weniger stark anisotropen Kristallitorientierung des anderen Bereichs.

Dieser Bereich mit vergleichsweise stärker anisotroper Kristallitorientierung, der federnd ist, ist erhältlich durch ein Walzverfahren zur Umformung eines metallischen Ausgangsformkörpers aus einer polykristallinen Stahllegierung gemäß Anspruch 4.

Bevorzugte Ausführungsformen des erfindungsgemässen Metallstreifens, seines Herstellungsverfahrens und der zugehörigen Vorrichtung ergeben sich aus den abhängigen Ansprüchen. Gegenstand der Erfindung sind auch die Walzvorrichtung zur Durchführung des erfindungsgemässen Verfahrens und Pinzetten, unterstützungsimplante und Gelenkprothesen, die unter Verwendung des erfindungsgemässen Verfahrens erhältlich sind.

Genaue Beschreibung der Erfindung

Die "Walzgeschwindigkeit v" in obiger Formel (1) ist die Geschwindigkeit, mit der sich ein gedachter Punkt, der auf der Drehachse der Walze in der Mitte zwischen den Durchstosspunkten der Drehachse durch die beiden Stirnflächen der Walze liegt, relativ zum Ausgangsformkörper vor seinem Eintritt in die Walzzone zwischen Walze und Unterlage bewegt.

Das erfindungsgemässe Verfahren wird so durchgeführt, dass an mindestens einer Stelle der Walzenoberfläche, die walzend mit dem Ausgangsformkörper in Berührung kommt, die Winkelgeschwindigkeit $\omega$ der Walze kleiner ist als v/R, wobei v und R die Bedeutung haben wie vorstehend definiert. Diese walzende Stelle kann diejenige Stelle sein, oder können diejenigen Stellen sein, deren Abstand R zur Drehachse im Vergleich zu den Abständen R der restlichen walzenden Stellen der Walzenoberfläche minimal ist oder sind. Bevorzugt ist es, wenn für jede walzende Stelle der Walzenoberfläche gilt, dass $\omega$ kleiner ist als v/R, wobei v und R die Bedeutung haben wie vorstehend definiert. Diese Merkmale unterscheiden sich von einem herkömmlichen Walzverfahren mit zwei gegenläufigen Walzen, wo für jede walzende Stelle der Walzenoberflächen beider Walzen gilt, dass die Winkelgeschwindigkeit $\omega$ der fraglichen Walze grösser ist als der Quotient v/R, wobei v und R die Bedeutung haben wie vorstehend definiert.

Die Winkelgeschwindigkeit m ist derjenige Winkel (im Bogenmass gemessen), den die Walze pro Zeiteinheit rotiert. Die Einheit der Winkelgeschwindigkeit $\omega$ ist also $s^{-1}$.

Bevorzugt beträgt die Winkelgeschwindigkeit $\omega$ 30 bis 95 % des Quotienten v/R, eher bevorzugt 50 bis 80%.

Die beim erfindungsgemässen Walzverfahren verringerte Winkelgeschwindigkeit $\omega$ kann wie folgt erzielt werden: Die Walze wird auf den zu walzenden, auf der Unterlage liegenden Ausgangsformkörper mit einer Normalkraft F gedrückt, die genügend gross ist, um den Ausgangsformkörper zu verformen. Die Walze wird dann in so angepresstem Zustand mit einer gewünschten Walzgeschwindigkeit v über den Ausgangsformkörper gestossen oder gezogen. Damit die Winkelgeschwindigkeit $\omega$ erfindungsgemäss kleiner bleibt als v/R, wird die Drehung der Walze gleichzeitig behindert oder gebremst. Durch die gebremste Rotation der Walze wird der Ausgangsformkörper nicht einfach flachgewalzt, sondern es baut sich beim erfindungsgemässen Verfahren vor der Walze ein gestauchter Materialwulst auf, der wie eine Druckwelle von der Walze hergeschoben wird.

Die besagte Behinderung oder Bremsung der Rotation der Walze kann einerseits mittels einer geeigneten Bremsvorrichtung, die während des Walzvorgangs auf die Walze einwirkt, erfolgen. Andererseits wirkt auch der besagte Materialwulst selber bremsend auf die Winkelgeschwindigkeit $\omega$ der Walze ein. Sofern die Parameter des Walzverfahrens wie Anpressdruck, Walzgeschwindigkeit usw. geeignet gewählt werden kann dann, sobald dieser Materialwulst entstanden ist, in günstigen Fällen auf eine explizite Bremsung der Walze verzichtet werden. Wenn eine Bremse verwendet wird, so kann dies jede Art von bekannter Bremse sein, so eine Reibungsbremse, wie etwa eine Trommelbremse, Scheibenbremse oder Keilbremse, eine Wirbelstrombremse oder eine hydraulisch betätigte oder wirkende Bremse (Viskositätsbremse). Die Steuerung der Winkelgeschwindigkeit der Walze kann auch bewirkt werden, indem ein elektrischer oder hydraulischer Motor vorgesehen wird, der an sich als Antrieb für die Walze wirken kann, aber dessen Drehzahl so gewählt wird, dass wiederum die besagte Steuerung der Winkelgeschwindigkeit der Walze bewirkt wird. Die Bremskraft kann mittels eines geeigneten Bremskraftreglers eingestellt oder reguliert werden. Die mindestens erforderliche Bremskraft ist dergestalt, dass die Rotation der Walze soweit gebremst wird, dass an mindestens einer walzenden Stelle der Walzenoberfläche $\omega$ < v/R wird. Die Bremskraft ist aber andererseits so gross, dass die Drehung der Walze teilweise oder gar ganz blockiert wird (d.h. $\omega \geq 0$ wird). Zwischen diesen beiden Grenzwerten kann die Bremskraft variiert werden und ergibt dann eine Winkelgeschwindigkeit $\omega$ der Walze, die kleiner ist als v/R und grösser gleich ist als Null.

Die Grösse des Zuges (Schubes) hängt vom Anpressdruck der Walze auf den Ausgangsformkörper, von der dadurch vor der Walze erzeugten Druckwelle und von der Walzgeschwindigkeit v ab. Der Anpressdruck muss dem gewünschten Umformgrad des Walzvorgangs entsprechen, muss aber unterhalb des Drucks bleiben, der zur Annäherung an oder zur Überschreitung der Streckgrenze des Materials des Ausgangsformkörpers führen würde. Die Grösse des vor der Walze hergeschobenen, bremsenden Materialwustes hängt direkt von der Grösse des Walzdruckes ab. Ihre Bremswirkung auf die Winkelgeschwindigkeit $\omega$ und die Walzgeschwindigkeit der Walze und somit auf den Zug (Schub) kann erhöht werden, indem die Walze als Profilwalze ausgebildet wird, was einen grösseren Kaltumformungsgrad bewirkt und somit der Walze eine grössere Arbeitsleistung abverlangt. Die Walzgeschwindigkeit v beeinflusst die Höhe des Materialwulstes mit den anderen Faktoren zusammen.

Bei dem erfindungsgemässen Verfahren kann die Walze in einer vorgegebenen Bewegung geradlinig oder gebogen kurvenförmig entsprechend dem gewünschten Profil über den zu walzenden Ausgangsformkörper geführt werden. Erfindungsgemäss bevorzugt wird die Walze dabei gestossen.

Das erfindungsgemässe Verfahren ist kein kontinuierlich arbeitendes Verfahren, da die Unterlage nur endliche Dimensionen hat und ein Walzzyklus spätestens nach Abrollen der Walze über die gesamte Unterlage beendet ist. Andererseits bietet es dadurch die Möglichkeit, einen Ausgangsformkörper in mehreren Zyklen hintereinander zu walzen, wobei diese Zyklen unmittelbar aufeinander abfolgen können, etwa immer mit der selben Walze, oder indem zwischen zwei aufeinanderfolgenden Zyklen die Walze ausgetauscht wird.

Die Walze muss erfindungsgemäss nicht zwingend eine Zylinderwalze sein; es kann auch eine Walze sein, die von der Zylinderform abweicht. Die Drehachse der Walze, von der aus auch die Abstände R zu den walzenden Stellen der Walzenoberfläche gemessen werden, muss nicht zwingend innerhalb der Walze liegen; bevorzugt ist sie aber innerhalb der Walze. Diese Abstände R sind grösser als Null. Erfindungsgemäss bevorzugt handelt es sich um eine Zylinderwalze oder um eine rotationssymmetrische Profilwalze oder um eine sektorielle Walze mit zylindrischer oder rotationssymmetrisch profilierter walzender Oberfläche. Bei einer Zylinderwalze, einer rotationssymmetrischen Profilwalze oder bei einer sektoriellen Walze mit zylindrischer oder rotationssymmetrisch profilierter Oberfläche fällt die Drehachse der Walze bevorzugt mit der Mittelachse der Walze zusammen. In den Fällen einer Zylinderwalze, einer sektoriellen Zylinderwalze, einer rotationssymmetrischen Profilwalze oder bei einer sektoriellen mit rotationssymmetrisch profilierter Oberfläche ist der besagte Abstand zwischen Drehachse und walzender Stelle der Walzoberfläche gerade gleich dem Radius R der Walze an dieser Stelle. Das gewünschtenfalls auf der Walzenoberfläche angebrachte Walzprofil ist bevorzugt dergestalt, dass die Basislinie seines Querschnittsprofils der Bandbreite des gewalzten Ausgangsformkörpers (d.h. des gewalzten Bereichs des erfindungsgemässen Metallstreifens) entspricht. Damit ein erfindungsgemässes Walzverfahren durchgeführt werden kann, d.h. höchstens eine unbedeutende Breitenzunahme auftritt, bleibt bei der Profilumformung für sich selber betrachtet die Querschnittsfläche des gewalzten Metallstreifens vorzugsweise konstant. Die Walze übernimmt beim erfindungsgemässen Verfahren gleichzeitig zwei Funktionen: 1) Die Pressfunktion zur Verringerung der Dicke des Ausgangsformkörpers, 2) die Zugfunktion zur Verlängerung des Walzgutes, was einer Verkleinerung des Querschnittes bei gleichbleibender oder nur unbedeutend zunehmender Breite entspricht.

Die Unterlage kann plan sein, sie kann auch eine geeignete gekrümmte oder in den drei räumlichen Dimensionen profilierte Oberfläche aufweisen. In diesem Fall wird der Ausgangsformkörper also nicht nur walzend umgeformt, sondern auch gleichzeitig formgewalzt. Vorzugsweise wird im Falle einer Profilierung der Oberfläche diese Profilierung ebenfalls so gewählt, dass sie zu einer für sich selber betrachtet querschnittskonstanten Umformung führt.

Beim erfindungsgemässen Verfahren wird auf eine explizite Zufuhr von Wärme verzichtet, so dass die einzige Wärmequelle, die auf den Ausgangsformkörper einwirkt, die im Formkörper selber während des Walzens entstehende Wärme ist. Dies bedeutet bevorzugt, dass die Temperatur des Ausgangsformkörpers und des entstehenden gewalzten Metallstreifens während des gesamten Walzvorgangs an keinem Ort den Wert von 100°C übersteigt.

Der Ausgangsformkörper, der mittels des erfindungsgemässen Verfahrens gewalzt werden kann, kann aus jedem genügend duktilen Metall bestehen. Wenn ein erfindungsgemässer Metallstreifen hergestellt werden soll ist der Ausgangsformkörper ausserdem aus einer polykristallinen Stahllegierung. Bevorzugt ist es eine nach dem kubisch innenzentrierten (bcc) Kristallgitter kristallisierende Stahllegierung, also etwa ein ferritischer, martensitischer oder gemischt ferritisch/martensitischer Stahl. Bei den martensitischen Stählen oder den gemischt ferritisch/martensitischen Stählen ist die $\alpha$'-Modifikation des Martensits bevorzugt. Diese kristallisiert streng genommen in einem tetragonal innenzentrierten Gitter, für praktische Zwecke kann sie ebenfalls als kubisch innenzentriert kristallisierend angenommen werden. Bevorzugt ist erfindungsgemäss ein nickel- und molybdänfreier martensitischer Stahl ("frei" heisst hier weniger als 0,01 Gewichtsprozent). Eher bevorzugt ist er von der Zusammensetzung Cr 12,50-14,50 Gewichtsprozent, C 0,42-0,50 Gewichtsprozent, Si max. 1,00 Gewichtsprozent, Mn max. 1,00 Gewichtsprozent, P max. 0,045 Gewichtsprozent, S max. 0,030 Gewichtsprozent, Rest im Wesentlichem Eisen und unvermeidbare Verunreinigungen. Insbesondere kann es ein Stahl gemäss der Werkstoff-Nr. 1.4034 sein.

Vorzugsweise hat der Ausgangsformkörper bereits selber die Form eines Metallstreifens oder Metallblechs.

Damit die walzende Verformung leichter und mit geringeren Drücken durchgeführt werden kann, kann der metallische Ausgangsformkörper, wenn er aus einer Stahllegierung besteht, vorgängig einer thermischen Behandlung, vorzugsweise mit anschliessender Abschreckung in kaltem Wasser, unterzogen werden. Diese Behandlung verringert isotrop die Festigkeit des Metalls. Auf dem Gebiet der Stahllegierungen ist diese thermische Behandlung als Lösungsglühung bekannt, wobei typisch ein Temperaturbereich von 1050-1080°C gewählt wird, und die Dauer der thermischen Behandlung typisch etwa 10 Minuten bis etwa 1 Stunde, bevorzugt etwa eine halbe Stunde beträgt.

Das erfindungsgemässe Verfahren eignet sich zur Herstellung aller Arten von Pinzetten, Nadelhaltern, Federn, Dissektoren, Klammern, Scheren (etwa Coiffeurscheren), Messern (wo das Walzen mit dem erfindungsgemässen Verfahren längs der Klingenachse anstatt wie bisher quer zur Klingenachse durchgeführt werden kann) oder Spezialprofilen jeglicher Art (etwa für den Fassadenbau oder den Rohrbau). Es eignet sich insbesondere für die Herstellung von Teilen, die mindestens einen federnden Bereich aufweisen, so für die Herstellung von Pinzetten, Nadelhaltern oder Federn, insbesondere auch für Pinzetten mit angesetzter vorwartswirkender oder rückwärtswirkender Schneidfunktion (Pinzettenscheren) oder für chirurgische und orthopädische Implantate.

Die erfindungsgemässe Vorrichtung, auf der das erfindungsgemässe Verfahren ausgeführt wird, umfasst mindestens eine Walze, eine Unterlage und eine Bremse wie vorstehend exemplifiziert, die während des Walzens zur Bremsung der Winkelgeschwindigkeit ω der Walze befähigt ist. Die Unterlage selber kann beweglich oder unbeweglich sein, bevorzugt ist, sie unbeweglich. Die erfindungsgemässe Vorrichtung umfasst des Weiteren die für das Anpressen und die Bewegung der Walze (Schub oder Zug) erforderlichen Lager und ihre Führungen. Diese Führungen der Walze umfassen bevorzugt hydraulische Zylinder oder mechanische Systeme, die in der Lage sind, die Walze präzise bei gleichbleibender oder gesteuerter Abstandskurve (gleich bleibende oder variierende Dicke des entstehenden gewalzten Metallstreifens) über die Unterlage zu führen. Diese hydraulischen oder mechanischen Führungen der Walze sind auf dem Gebiet des Walzens bekannt und bedürfen keiner Erläuterung. Durch die geeignet gesteuerte Bremsung der Walze und ihr gleichzeitiges Vorwärtsziehen oder -stossen erübrigt sich bei der erfindungsgemässen Vorrichtung eine zusätzliche Ziehvorrichtung für das Walzgut.

Als Ergebnis des erfindungsgemässen Verfahrens und weiterer fakultativer, vorgängiger Umformungsschritte entsteht ein mindestens teilweise gewalzter Metallstreifen. Dieser Metallstreifen hat nicht zwingend eine regelmässige oder plane Form. Er besteht aus einem Stück, das heisst, dass er nicht aus zwei oder mehreren Einzelteilen verschraubt, vernietet oder verklebt ist. Er weist des Weiteren keine Schweissstelle auf.

Allgemein weist ein erfindungsgemässer Streifen aus einem polykristallinen Metall, der einen wie vorstehend beschrieben gewalzten Bereich umfasst, in diesem Bereich eine vergleichsweise stärker anisotrope Orientierung der Kristallite auf. In dem oder den nicht gewalzten Bereichen weist er die Kristallitorientierung des Ausgangsformkörpers auf, d.h. also eine vergleichsweise geringer anisotrope oder unter Umständen im Wesentlichen isotrope Orientierung der Kristallite. Der Begriff "nicht gewalzt" umfasst auch eine Vorbehandlung des Ausgangsformkörpers unter Walzen, sofern als letzter Schritt dieser Vorbehandlung eine Wärmebehandlung wie vorstehend beschrieben durchgeführt wurde, die allfällige Veränderungen des Gitters, die durch das Walzen verursacht sein konnten, wieder rückgängig macht.

Die Orientierung der Kristallite wird im Rahmen der vorliegenden Erfindung über die Orientierungsdichtefunktion, in der Fachsprache auch als ODF abgekürzt, definiert. Im Englischen wird sie als "orientational distribution function", ebenfalls abgekürzt ODF, bezeichnet.

Im Rahmen der hiesigen Anmeldung wird für den gewalzten Streifen ein orthogonales Koordinatensystem angenommen, dessen X-Achse parallel zur Walzrichtung des erfindungsgemäss gewalzten Bereichs des Streifens ist; dessen Y-Achse in Querrichtung zur Walzrichtung ist und, wenn der Streifen von seiner Oberseite betrachet wird, nach links zeigt, und dessen Z-Achse die Normale als Vektorprodukt aus X-Achse und Z-Achse ist.

Die besagte Orientierungsdichtefunktion ODF wird im Rahmen der vorliegenden Anmeldung aus mit CuKα-Strahlung (λ = 1,54 Angström) erhaltenen Röntgenstrukturdaten bestimmt. Es können einerseits beispielsweise scheibenförmige Proben verwendet werden, die aus den zu untersuchenden Streifen herausgeschnitten werden, und die eine plane, zu bestrahlende Oberfläche aufweisen. Die zu untersuchende Probe wird dabei so aus dem Streifen herausgeschnitten, dass die besagte zu bestrahlende Oberfläche senkrecht auf der Z-Achse steht. Sofern der Streifen bereits eine plane Oberfläche aufweist, die senkrecht auf der Z-Achse steht, könnte auch der Streifen direkt an dieser Oberfläche vermessen werden.

Diese ODF kann einerseits als

$$f(\varphi_1, \Phi, \varphi_2) = \frac{dV}{d\Phi d\varphi_1 d\varphi_2} \cdot \frac{8\pi^2}{\sin \Phi} \qquad (2a)$$

definiert werden. In Formel (2a) bedeuten:

$\varphi_1$, $\Phi$, $\varphi_2$:    Die drei Eulerwinkel, die die Drehung des kristallitinternen Koordinatensystems gegenüber dem Probenkoordinatensystem beschreiben. Das kristallitinterne Koordinatensystem ist für jeden einzelnen Kristalliten individuell.

$dV/d\Phi\varphi_1 d\varphi_2$:    Der differentielle Volumenanteil $dV$ derjenigen Kristallite, bei denen das kristallitinterne Koordinatensystem eine Orientierung innerhalb eines differentiellen, gegebenen Raumwinkelanteils hat (beschrieben mit den differentiellen Eulerwinkeln $d\Phi$, $d\varphi_1$, $d\varphi_2$).

V:    Das gesamte Volumen aller bestrahlten Kristallite.

Die Bestimmung dieser ODF als f ($\varphi_1$, $\Phi$, $\varphi_2$) kann aus Polfigurenmessungen vorgenommen werden. Die Polfiguren und die ODF werden als Reihenentwicklungen von verallgemeinerten Kugelfunktionen approximiert, diese beiden Approximationen werden in die Fundamentalgleichung der Texturanalyse eingesetzt, und die Koeffizienten der Reihenentwicklung daraus bestimmt. Diese Vorgehensweise ist in den Abschnitten 11.4.1 und 11.6.5 ("harmonische Methode") des Lehrbuchs "Moderne Röntgenbeugung", L. Spiess, R. Schwarzer, H. Behnken, G. Teichert, Oktober 2005, B.G. Teubner Verlag, Wiesbaden, Deutschland, beschrieben.

Die ODF kann andererseits auch mittels der Formel (2b):

$$W(\vartheta,\varphi) \equiv \frac{dV}{d\Omega}\frac{4\pi}{V} \quad (2b)$$

definiert werden. In Formel (2b) bedeuten:

$dV/d\Omega$ : Der differentielle Volumenanteil $dV$ derjenigen Kristallite, bei denen die Flächennormalen der bestrahlten Oberfläche der Probe (siehe unten) innerhalb eines differentiellen, gegebenen Raumwinkelanteils $d\Omega$ im kristallitinternen Koordinatensystem (siehe seine Beschreibung bei obiger Formel (2a)) zu liegen kommt.

V: Das gesamte Volumen aller bestrahlten Kristallite.

Diese Orientierungsdichtefunktion $W(\vartheta,\varphi)$ weist als Variablen einen von den z-Achsen der kristallitinternen Koordinatensysteme aus gemessenen Polarwinkel $\vartheta$ und einen von den x-Achsen aus gemessenen Azimutalwinkel $\varphi$ auf. Diese ODF wird gemäss den folgenden Schritten a)-c) (Siehe auch J. Appli. Cryst. 1970, 3, p. 313ff.) erhalten:

a) Die Probe wird auf dem Probenhalter des Diffraktogramms fixiert, so dass die Flächennormale der zu bestrahlenden Oberfläche senkrecht zur Diffraktometerachse ist. Man wählt den Goniometer-Winkel 2θ dergestalt, dass eine Diffraktion an einer Kristallgitterebenenschar mit einem bestimmten Miller-Index (*hkl*) detektiert wird. Die Probe (bzw. der Streifen selber) wird dann so geneigt, dass die Flächennormale der zu bestrahlenden Oberfläche um einen Winkel $\alpha$ von der Normalen dieser Ebenenschar zur Diffraktometerachse hin gedreht wird. Mit dieser Neigung, unter gleichzeitiger Rotation der Probe um die Flächennormale der zu bestrahlenden Oberfläche um 360° und unter Beibehalt des vorgängig gewählten Goniometerwinkels 2θ, wird die summierte Diffraktionsintensität $I_{hkl}(\alpha)$ gemessen. Diese Messung wird für insgesamt K verschiedene Winkel $\alpha$, aber mit stets demselben θ durchgeführt.

b) Für jede in a) bestimmte $I_{hki}(\alpha)$ wird wiederum eine Reihenentwicklung der Form

$$I_{hkl}(\alpha) = A(h,k,l,\theta,\lambda)\left(1 + \sum_{v}^{V}\sum_{w}^{W} C_{vw}K_{vw}(\vartheta_{hkl},\varphi_{hkl})P_{v}(\cos\alpha)\right) \quad (3)$$

angesetzt. Darin ist $K_{vw}(\vartheta_{hkl},\varphi_{hkl})$ der Wert der an die Kristallgittersymmetrie des fraglichen Metalls adaptierte Kugelfunktion $K_{vw}$ (symmetrieadaptierte Kugelfunktion, englisch "symmetry-adapted spherical harmonic", SASH) bei dem Winkelpaar $(\vartheta,\varphi)$, das die Richtung des Normalvektors der Kristallebenenschar mit Miller-Index *(hkl)* in den kristallitinternen Koordinatensystemen angibt. Der Index v läuft nur über die geraden Zahlen grösser Null bis zur maximal berücksichtigten Anzahl V. Je grösser V, desto höher die Genauigkeit. Der Index w läuft über alle linear unabhängigen solchen Kugelfunktionen innerhalb der Ordnung v. Die Anzahl K der in a) gemessenen $I_{hkl}(\alpha)$ muss um eins grösser sein als die Gesamtzahl der Summanden in der Doppelsumme von Formel (3). $P_{v}(\cos\alpha)$ ist der Wert des Legendre-Polynoms der Ordnung v bei $\cos\alpha$. Des Weiteren ist in Formel (3):

$$A(h,k,l,\theta,\lambda) = \frac{Q}{2\mu}\left|\sum_{j=1}^{N} f_{j}\left(\frac{\sin(\theta)}{\lambda}\right)\exp(-2i\pi(hx_{j}+ky_{j}+lz_{j}))\right|^{2} \frac{P_{hkl}(1+\cos^{2}(2\theta))}{8\sin^{2}(\theta)\cos(\theta)}\exp\left(B\frac{\sin^{2}(\theta)}{\lambda^{2}}\right) \quad (4)$$

worin bedeuten:

Q: Eine für alle Reflektionen gleiche Konstante.

$\mu$: Der lineare Absorptionskoeffizient des untersuchten Metalls für die CuKα-Strahlung. Diese Absorptionskoeffizienten sind bekannt.

$f_j(\dfrac{\sin(\theta)}{\lambda})$ :     Der Atomformfaktor des j-ten Atoms der Elementarzelle, in Abhängigkeit von $\sin(\theta)/\lambda$. $\lambda$ ist 1,54 Angström. Diese Atomformfaktoren sind bekannt. Die Summe, worin diese Atomformfaktoren vorkommen, läuft über alle N Atome der Elementarzelle.

$x_j,\ y_j,\ z_j$:     Die Koordinaten des j-ten Atoms in der Elementarzelle in kristallitinternen Koordinaten (siehe oben).

$P_{hkl}$:     Die Multiplizität der detektierten Intensität, d.h. die Anzahl der äquivalenten Kristallebenenscharen, die zu $I_{hkl}(\alpha)$ beitragen. Diese Multiplizitäten sind für alle Arten von Kristallgittern bekannt.

$B$:     Der näherungsweise als isotrop und für alle Atome der Elementarzelle gleich angenommene Temperaturfaktor. Diese Temperaturfaktoren sind bekannt. Aus den Gleichungen (3) werden wiederum die Koeffizienten $C_{vw}$ und auch das Q bestimmt.

c) Die Orientierungsdichtefunktion $W(\vartheta,\varphi)$ wird mittels der in b) erhaltenen Koeffizienten $C_{vw}$ und mittels der Formel

$$W(\vartheta,\varphi) = 1 + \sum_{v}^{V} \sum_{w}^{W} C_{vw} K_{vw}(\vartheta,\varphi) \quad (5)$$

erhalten, worin $K_{vw}(\vartheta,\varphi)$ wiederum die oben erwähnten symmetrieadaptierten Kugelfunktionen sind, das Winkelpaar $(\vartheta,\varphi)$ innerhalb des kristallitinternen Koordinatensystems wie vorstehend beschrieben ist, und v, V, w und W die oben angegebene Bedeutung haben.

Die Orientierung der Kristallite im erfindungsgemäss gewalzten Bereich ist stärker anisotrop als in einem anderen Bereich, der nicht gewalzt oder herkömmlich gewalzt ist. Vorzugsweise ist die Anisotropie der Kristallitorientierung in dem Bereich, wo diese Anisotropie vergleichsweise stärker ist, dergestalt, dass die oben beschriebene ODF gemäss Formel (2b) in ihrer approximativen Reihenentwicklung mindestens ein $C_{vw}$ enthält, das betragsmässig mindestens 0,050 ist; eher bevorzugt ist dieses $C_{vw}$ betragsmässig mindestens 0,100 und noch eher bevorzugt mindestens 0,200. Demgegenüber ist die oben beschriebene ODF gemäss (2b) in dem Bereich mit vergleichsweise weniger stark anisotroper Kristallitorientierung bevorzugt dergestalt, dass in der besagten Reihenentwicklung der ODF keines der $C_{vw}$ betragsmässig grösser ist als 0,050, d.h. dass sie im Wesentlichen isotrop ist (für eine exakt isotrope Kristallitorientierung wären alle $C_{vw}$ Null).

Im Falle von Streifen, die sowohl einen erfindungsgemäss gewalzten Bereich als auch einen nicht gewalzten Bereich aufweisen, und die aus einem ferritischer, martensitischer oder gemischt ferritisch/martensitischer Stahl wie oben beschrieben, bestehen, äussert sich diese Anisotropie der Orientierung speziell wie folgt: Wenn man an einem solchen Streifen θ-2θ-Diffraktogramme von Proben des gewalzten und des nicht gewalzten Bereichs dergestalt aufnimmt, dass die Diffraktometerachse parallel zu der zu bestrahlenden Oberfläche der Probe ist, so wird gefunden, dass die Kristallite im erfindungsgemäss gewalzten Bereich vergleichsweise häufiger so orientiert sind, dass ihre Ebenenschar mit Miller-Index (200) parallel zur bestrahlten Oberfläche ist. In geringerem Ausmass sind die Kristallite auch so orientiert, dass ihre Ebenenschar mit Miller-Index (211) häufiger parallel zur bestrahlten Oberfläche ist. Diese besondere Orientierung der Kristallite wird von den Randzonen des erfindungsgemäss gewalzten Bereichs (von aussen) zu seiner Mitte hin (nach innen) ausgeprägter. Demgegenüber beobachtet man an einem Streifen aus demselben Metall, der aber einen nach einem herkömmlichen Verfahren (mit zwei Walzen) gewalzten Bereich umfasst, keine solche besondere Orientierung.

Ebenfalls allgemein zeigen Streifen, die aus einem polykristallinen Metall, bevorzugt einem im kubisch innenzentrierten (bcc) Kristallgitter kristallisierenden Metall, eher bevorzugt aus einem ferritischen, martensitischen oder gemischt ferritisch/martensitischen Stahl wie oben beschrieben, bestehen (insbesondere gemäss der Werkstoff-Nr. 1.4034), und die einen erfindungsgemäss gewalzten Bereich und einen nicht gewalzten Bereich umfassen, Folgendes: Wenn sie nach dem im vorangehenden Paragraph beschriebenen Diffraktionsverfahren an zwei beliebigen Stellen untersucht werden, so werden keine statistisch signifikanten Unterschiede in der Lage der Piken oder in ihrer Form gefunden, wenn die entsprechenden Piken der beiden Diffraktogramme miteinander verglichen werden, d.h. die beiden Piken sind hinsichtlich Lage und Pikenform nie statistisch signifikant voneinander verschieden. Dies ist wiederum im Unterschied zu einem Streifen aus demselben Metall, der aber einen Bereich umfasst, der nach einem herkömmlichen Verfahren (mit zwei Walzen) gewalzt ist: Dort kann die Pike aus einem Diffraktogramm des gewalzten Bereichs gegenüber der entsprechenden Pike aus einem Diffraktogramm des nicht gewalzten Bereichs statistisch signifikant in ihrer Lage verschoben und/oder in ihrer Form statistisch signifikant verzerrt sein.

Im Rahmen der vorliegenden Anmeldung werden die Symmetrie einer Pike betreffend ihres Maximums und die Schärfe einer Pike (Verhältnis von Halbwertsbreite zu Maximalintensität) als "Form" dieser Pike verstanden. Nicht als "Form" der Pike wird ihre Intensität verstanden.

Ob zwei Piken "hinsichtlich Lage oder Form statistisch signifikant voneinander verschieden" sind, wird im Rahmen der vorliegenden Anmeldung gemäss folgenden Schritten 1)-4) festgestellt:

1) Man stellt die beiden zu vergleichenden Diffraktogramme in einer Form bereit, in der die absoluten Intensitätskurven der zu vergleichenden Piken als Funktion von 28 in diskreten Zählintervallen der Breite 0,05° registriert sind. Jedes dieser Zählintervalle wird im Folgenden über einen zugehörigen Index i bezeichnet.

2) Man sucht sich in den beiden Diffraktogrammen den grösstmöglichen zusammenhängenden und übereinstimmenden 2θ-Bereich aus, der die Maxima der beiden zu vergleichenden Piken enthält und in dem immer entweder die absolute Intensität des i-ten Zählintervalls aus dem ersten Diffraktogramm mindestens doppelt so gross ist wie der zugehörige Untergrund, oder die absolute Intensität des entsprechenden i-ten Zählintervalls des zweiten Diffraktogramms mindestens doppelt so gross ist wie der zugehörige Untergrund, oder gar beide i-ten Intensitäten mindestens doppelt so gross sind wie der jeweils zugehörige Untergrund. Existiert kein solcher Bereich, werden die beiden Piken als "hinsichtlich Lage statistisch signifikant voneinander verschieden" bewertet und der Rest des Tests wird nicht mehr durchgeführt.

3) Sofern aber ein solcher Bereich mit insgesamt k zusammenhängenden Zählintervallen existiert, wird aus allen diesen Zählintervallen die Kenngrösse Chi-Quadrat berechnet:

$$X^2 = \sum_{i=1}^{k} \frac{({}_1b_i - {}_2b_i)^2}{2({}_1b_i + {}_2b_i)} \quad (6)$$

In dieser Formel sind ${}_1b_i$ bzw. ${}_2b_i$ die untergrundbereinigten und dann auf ein Pikenmaximum von 100 (hundert) Zählimpulse normierten Intensitäten der ersten bzw. zweiten Pike im i-ten Zählintervall. Die Normierung der zu vergleichenden Piken auf ein Maximum von 100 Zählimpulsen geschieht erstens deswegen, weil die Anzahl der bestrahlten Kristallite von Probenort zu Probenort nicht konstant ist (dies führt zu unterschiedlichen Intensitäten bei den an beiden Probenorten aufgenommen Diffraktogrammen), und zweitens, weil Unterschiede in der Anisotropie der Kristallitorientierung vorhanden sein können (dies führt zu Variationen der Intensitätsverhältnisse der Piken innerhalb eines Diffraktogramms; insbesondere werden bei einem erfindungsgemässen Streifen ja mindestens ein Bereich mit vergleichsweise stärker anisotroper Kristallitorientierung und mindestens ein Bereich mit vergleichsweise weniger stark anisotroper Kristallitorientierung gefordert). Der hier beschriebene statistische Test soll aber losgelöst und unabhängig von der Anisotropie der Kristallitorientierung lediglich statistisch signifikante Unterschiede in den Eigenschaften der Kristallite selber zeigen, z.B. eine geringere mittlere Grösse (ersichtlich als Pikenverbreiterung) oder Spannungen innerhalb des Kristallgitters (ersichtlich als Piken-Maximumsverschiebung, Pikenverbreiterung oder Asymmetrie in der Pikenform). Obige Formel (6) ergibt sich aus der in der Mathematik üblichen Formel für die Chi-Quadrat-Testgrösse für beobachtete Zählintensitäten ${}_1b_i$ oder ${}_2b_i$, sofern für den zugehörigen i-ten Erwartungswert $\mu_i$ der Mittelwert aus ${}_1b_i$ und ${}_2b_i$ und für die Standardabweichung $\sigma_i$ die Quadratwurzel aus diesem Mittelwert eingesetzt wird.

4) Man vergleicht die in Schritt 3) berechnete Chi-Quadrat-Kenngrösse mit dem Wert der Chi-Quadrat-Verteilung bei k Freiheitsgraden gemäss der folgenden Tabelle 1 bei einer Signifikanzschranke von 0,001% (k ist die Anzahl der Zählintervalle des unter Schritt 2) ermittelten zusammenhängenden Bereichs):

Tabelle 1

| Freiheitsgrade k | Signifikanzschranke | | | | | |
|---|---|---|---|---|---|---|
| | 0,001% | 0,01% | 0,1% | 1% | 5% | 10% |
| 2 | 23,03 | 18,42 | 13,82 | 9,21 | 5,99 | 4,61 |
| 3 | 25,75 | 20,97 | 16,14 | 11,24 | 7,74 | 6,19 |
| 4 | 28,47 | 23,51 | 18,47 | 13,28 | 9,49 | 7,78 |
| 5 | 30,79 | 25,68 | 20,46 | 15,04 | 11,04 | 9,21 |
| 6 | 33,11 | 27,86 | 22,46 | 16,81 | 12,59 | 10,64 |
| 7 | 35,22 | 29,84 | 24,29 | 18,45 | 14,05 | 12,00 |
| 8 | 37,33 | 31,83 | 26,12 | 20,09 | 15,51 | 13,36 |
| 9 | 39,31 | 33,70 | 27,86 | 21,65 | 16,91 | 14,67 |
| 10 | 41,30 | 35,56 | 29,59 | 23,21 | 18,31 | 15,99 |

(fortgesetzt)

| Freiheitsgrade k | Signifikanzschranke | | | | | |
|---|---|---|---|---|---|---|
| | 0,001% | 0,01% | 0,1% | 1% | 5% | 10% |
| 11 | 43,19 | 37,35 | 31,25 | 24,71 | 19,67 | 17,27 |
| 12 | 45,08 | 39,13 | 32,91 | 26,22 | 21,03 | 18,55 |
| 13 | 46,90 | 40,86 | 34,52 | 27,68 | 22,36 | 19,81 |
| 14 | 48,72 | 42,58 | 36,12 | 29,14 | 23,68 | 21,06 |
| 15 | 50,48 | 44,25 | 37,69 | 30,57 | 24,99 | 22,30 |
| 16 | 52,24 | 45,92 | 39,25 | 32,00 | 26,30 | 23,54 |
| 17 | 53,96 | 47,56 | 40,78 | 33,40 | 27,58 | 24,77 |
| 18 | 55,68 | 49,19 | 42,31 | 34,81 | 28,87 | 25,99 |
| 19 | 57,36 | 50,79 | 43,81 | 36,19 | 30,14 | 27,20 |
| 20 | 59,04 | 52,39 | 45,31 | 37,57 | 31,41 | 28,41 |
| 21 | 60,69 | 53,96 | 46,79 | 38,93 | 32,67 | 29,61 |
| 22 | 62,34 | 55,52 | 48,27 | 40,29 | 33,92 | 30,81 |
| 23 | 63,96 | 57,07 | 49,72 | 41,63 | 35, 17 | 32,00 |
| 24 | 65, 58 | 58,61 | 51,18 | 42,98 | 36,42 | 33,20 |
| 25 | 67,18 | 60,14 | 52,62 | 44,31 | 37, 65 | 34,38 |
| 26 | 68,77 | 61,66 | 54,05 | 45,64 | 38,89 | 35,56 |
| 27 | 70,34 | 63,16 | 55,47 | 46,96 | 40,11 | 36,74 |
| 28 | 71,92 | 64,66 | 56,89 | 48,28 | 41,34 | 37, 92 |
| 29 | 73,47 | 66,15 | 58,30 | 49,59 | 42,56 | 39,09 |
| 30 | 75,02 | 67,63 | 59,70 | 50,89 | 43,77 | 40,26 |
| 31 | 76,56 | 69,10 | 61,10 | 52,19 | 44,98 | 41,42 |
| 32 | 78,09 | 70,57 | 62, 49 | 53,49 | 46,19 | 42,58 |
| 33 | 79,61 | 72,03 | 63,87 | 54,77 | 47,40 | 43,74 |
| 34 | 81,13 | 73,48 | 65,25 | 56,06 | 48,60 | 44,90 |
| 35 | 82,64 | 74,92 | 66,62 | 57,34 | 49,80 | 46,06 |
| 36 | 84,14 | 76,36 | 67,99 | 58,62 | 51,00 | 47,21 |
| 37 | 85,63 | 77,79 | 69,34 | 59,89 | 52,19 | 48,36 |
| 38 | 87,12 | 79,22 | 70,70 | 61,16 | 53,38 | 49,51 |
| 39 | 88,60 | 80,64 | 72,05 | 62, 43 | 54,57 | 50,66 |
| 40 | 90,08 | 82,06 | 73,40 | 63,69 | 55,76 | 51,81 |
| 41 | 91,55 | 83,47 | 74,74 | 64,95 | 56,94 | 52,95 |
| 92 | 93,01 | 84,88 | 76,08 | 66,21 | 58,12 | 54,09 |
| 43 | 94,47 | 86,28 | 77,92 | 67, 46 | 59,30 | 55,23 |
| 44 | 95,92 | 87,68 | 78,75 | 68,71 | 60,48 | 56,37 |
| 45 | 97,37 | 89,07 | 80,07 | 69,96 | 61,66 | 57,50 |
| 46 | 98,81 | 90,46 | 81,40 | 71,20 | 62,83 | 58, 64 |
| 97 | 100,25 | 91,84 | 82,72 | 72,44 | 64,00 | 59,77 |

(fortgesetzt)

| Freiheitsgrade k | Signifikanzschranke | | | | | |
|---|---|---|---|---|---|---|
| | 0,001% | 0,01% | 0,1% | 1% | 5% | 10% |
| 48 | 101,69 | 93,22 | 84,04 | 73,68 | 65,17 | 60,91 |
| 49 | 103,11 | 94,59 | 85,35 | 74,92 | 66,34 | 62,04 |
| 50 | 104,54 | 95,97 | 86,66 | 76,15 | 67,50 | 63,17 |
| 51 | 105,96 | 97,34 | 87,97 | 77,38 | 68,67 | 64,29 |
| 52 | 107,38 | 98,70 | 89,27 | 78,62 | 69,83 | 65,42 |
| 53 | 108,79 | 100,06 | 90,57 | 79,84 | 70,99 | 66,55 |
| 54 | 110,20 | 101,42 | 91,87 | 81,07 | 72,15 | 67,67 |
| 55 | 111,61 | 102,77 | 93,17 | 82,29 | 73,31 | 68,80 |
| 56 | 113,01 | 104,13 | 94,46 | 83,51 | 74,47 | 69,92 |
| 57 | 114,41 | 105,47 | 95,75 | 84,73 | 75,62 | 71,04 |
| 58 | 115,80 | 106,82 | 97,04 | 85,95 | 76,78 | 72,16 |
| 59 | 117,19 | 108,16 | 98,32 | 87,16 | 77,93 | 73,28 |
| 60 | 118,58 | 109,50 | 99,61 | 88,38 | 79,08 | 74,90 |
| 61 | 119,96 | 110,84 | 100,89 | 89,59 | 80,23 | 75,51 |
| 62 | 121,35 | 112,17 | 102,17 | 90,80 | 81,38 | 76,63 |
| 63 | 122,73 | 113,50 | 103,44 | 92,01 | 82,53 | 77,74 |
| 64 | 124,10 | 114,83 | 104,72 | 93,22 | 83,68 | 78,86 |
| 65 | 125,47 | 116,16 | 105,99 | 94,42 | 84,82 | 79,97 |
| 66 | 126,85 | 117,48 | 107,26 | 95,63 | 85,96 | 81,09 |
| 67 | 128,21 | 118,80 | 108,52 | 96,83 | 87,11 | 82,20 |
| 68 | 129,58 | 120,12 | 109,79 | 98,03 | 88,25 | 83,31 |
| 69 | 130,94 | 121,44 | 111,05 | 99,23 | 89,39 | 84,42 |
| 70 | 132,30 | 122,75 | 112,32 | 100,43 | 90,53 | 85,53 |
| 71 | 133,66 | 124,07 | 113,58 | 101,62 | 91,67 | 86,64 |
| 72 | 135,01 | 125,38 | 114,84 | 102,82 | 92,81 | 87,74 |
| 73 | 136,36 | 126,68 | 116,09 | 104,01 | 93,94 | 88,85 |
| 74 | 137,71 | 127,99 | 117,35 | 105,20 | 95,08 | 89,96 |
| 75 | 139,06 | 129,29 | 118,60 | 106,39 | 96,22 | 91,06 |
| 76 | 140,41 | 130,60 | 119,85 | 107,58 | 97,35 | 92,17 |
| 77 | 141,75 | 131,89 | 121,10 | 108,77 | 98,48 | 93,27 |
| 78 | 143,09 | 133,19 | 122,35 | 109,96 | 99,62 | 94,37 |
| 79 | 144,43 | 134,49 | 123,59 | 111,14 | 100,75 | 95,48 |
| 80 | 145,76 | 135,78 | 124,84 | 112,33 | 101,88 | 96,58 |
| 81 | 147,10 | 137,07 | 126,08 | 113,51 | 103,01 | 97,68 |
| 82 | 148,43 | 138,37 | 127,32 | 114,69 | 104,14 | 98,78 |
| 83 | 149,76 | 139,65 | 128,56 | 115,88 | 105,27 | 99,88 |
| 84 | 151,09 | 140,94 | 129,80 | 117,06 | 106,39 | 100,98 |

(fortgesetzt)

| Freiheitsgrade k | Signifikanzschranke | | | | | |
|---|---|---|---|---|---|---|
| | 0,001% | 0,01% | 0,1% | 1% | 5% | 10% |
| 85 | 152,41 | 142,22 | 131,04 | 118,24 | 107,52 | 102,08 |
| 86 | 153,74 | 143,51 | 132,28 | 119,41 | 108,65 | 103,18 |
| 87 | 155,06 | 144,79 | 133,51 | 120,59 | 109,77 | 104,27 |
| 88 | 156,38 | 146,07 | 134,75 | 121,77 | 110,90 | 105,37 |
| 89 | 157,70 | 147,35 | 135,98 | 122,94 | 112,02 | 106,47 |
| 90 | 159,02 | 148,63 | 137,21 | 124,12 | 113,15 | 107,57 |
| 91 | 160,33 | 149,90 | 138,44 | 125,29 | 114,27 | 108,66 |
| 92 | 161,65 | 151,18 | 139,67 | 126,46 | 115,39 | 109,76 |
| 93 | 162,96 | 152,45 | 140,89 | 127,63 | 116,51 | 110,85 |
| 94 | 164,27 | 153,72 | 142,12 | 128,80 | 117,63 | 111,94 |
| 95 | 165,58 | 154,99 | 143,34 | 129,97 | 118,75 | 113,04 |
| 96 | 166,89 | 156,26 | 144,57 | 131,14 | 119,87 | 114,13 |
| 97 | 168,19 | 157,53 | 145,79 | 132,31 | 120,99 | 115,22 |
| 98 | 169,50 | 158,79 | 147,01 | 133,48 | 122,11 | 116,32 |
| 99 | 170,80 | 160,06 | 148,23 | 134,64 | 123,22 | 117,41 |
| 100 | 172,10 | 161,32 | 149,45 | 135,81 | 124,34 | 118,50 |

Wenn die in Schritt 3) berechnete Chi-Quadrat-Kenngrösse grösser ist als der in Tabelle 1 bei der anwendbaren Zahl Freiheitsgrade k aufgeführte Wert, sind die beiden Piken "hinsichtlich Lage oder Form statistisch signifikant voneinander verschieden", andernfalls sind sie "hinsichtlich Lage und Form statistisch nicht signifikant voneinander verschieden".

Vorzugsweise ist diese Chi-Quadrat-Kenngrösse aus zwei entsprechenden Piken von zwei Diffraktogrammen von zwei beliebigen Stellen eines mindestens teilweise erfindungsgemäss gewalzten Streifens, aber nirgendwo herkömmlich gewalzten Streifens immer so klein, dass die beiden Piken selbst dann noch als "hinsichtlich Lage und Form statistisch nicht signifikant voneinander verschieden" erkannt werden, wenn aus der obigen Tabelle nicht die Werte bei der Signifikanzschranke 0,001%, sondern (in ansteigender Bevorzugung) bei 0,01%, 0,1%, 1%, 5% oder 10% verwendet werden.

Im Fall eines Metalls, das in einem innenzentrierten, insbesondere kubisch innenzentrierten Gitter kristallisiert (darunter die bevorzugten ferritischen, martensitischen oder gemischt ferritisch/martensitischen Stähle), oder in einem flächenzentrierten Gitter, insbesondere einem kubisch flächenzentrierten Gitter kristallisiert (etwa die austenitischen Stähle), werden statistisch signifikante Unterschiede zwischen den beiden Diffraktogrammen am ehesten beim Vergleichen der Piken mit Miller-Index (200) beobachtet.

Nach dem oben beschriebenen statistischen Test wurden mit einem Computerprogramm beispielsweise Diffraktogramme von zwei Streifen A und B aus einem Stahl der Werkstoff-Nr. 1.4034, von 70 mm Länge und 10 mm Breite untersucht, die an einem Ende nicht gewalzt sind und eine konstante Dicke von etwa 1,5 mm aufwiesen und am anderen Ende auf eine konstante Dicke von etwa 0,85 mm entweder erfindungsgemäss gewalzt waren (Streifen A) oder herkömmlich gewalzt waren (Streifen B). Auf statistisch signifikante Unterschiede getestet wurde die Pike bei $2\theta$ = etwa 64,8° (die (200)-Pike). Das Programm erforderte die manuelle Eingabe der Lage der Pikenmaxima und links und rechts der beiden Piken je ein Untergrundgebiet. Als Untergrund berechnete das Programm den Mittelwert aller Intensitäten aus diesen beiden Untergrundgebieten. Das Programm ermittelte dann den grösstmöglichen zusammenhängenden $2\theta$-Bereich gemäss obigem Schritt 2) und die Anzahl der Freiheitsgrade k, und berechnete den Chi-Quadratwert nach obiger Formel (6). Es wurden folgende Chi-Quadrat-Werte und zugehörige Freiheitsgrade k erhalten:

Tabelle 2a (Streifen A)

| Messort des 1. Diffraktogramms | Messort des 2. Diffraktogramms | Chi-Quadrat nach Formel (6) (vier Berechnungen mit verschieden gewähltem Untergrund) | Freiheitsgrade (= Anzahl berück-sichtigte Zählin-tervalle des zusammenhängenden 2θ-Bereichs) | Sind die Piken bei 64,8° aus 1. und 2. Diffraktogramm bei einer Signifikanzschranke von 0,001% "hinsichtlich Lage oder Form statistisch signifikant voneinander verschieden"? Ja / Nein |
|---|---|---|---|---|
| Rückseite des gewalzten Bereichs, in Walzrichtung | Rückseite des gewalzten Bereichs, in Walzrichtung | 0,014<br>0,003<br>0,008<br>0,028 | 17<br>17<br>17<br>17 | Nein |
| Rückseite des gewalzten Bereichs, in Walzrichtung | Oberseite des gewalzten Bereichs, in Walzrichtung | 6,466<br>6,712<br>6,448<br>6,554 | 18<br>18<br>18<br>18 | Nein |
| Rückseite des gewalzten Bereichs, in Walzrichtung | Oberseite des nicht gewalzten Bereichs, in Walzrichtung | 2,924<br>3,133<br>2,985<br>3,104 | 18<br>18<br>18<br>18 | Nein |
| Rückseite des gewalzten Bereichs, in Walzrichtung | Rückseite des gewalzten Bereichs, quer zur Walzrichtung, äusserster Randbereich | 6,865<br>7,416<br>6,876<br>7,648 | 23<br>23<br>23<br>23 | Nein |
| Rückseite des gewalzten Bereichs, in Walzrichtung | Rückseite des gewalzten Bereichs, quer zur Walzrichtung, innerer Randbereich | 6,325<br>6,551<br>6,685<br>6,453 | 25<br>25<br>25<br>25 | Nein |
| Rückseite des gewalzten Bereichs, in Walzrichtung | Rückseite des gewalzten Bereichs, quer zur Walzrichtung, Mitte | 11,196<br>11,200<br>11,188<br>11,192 | 27<br>27<br>27<br>27 | Nein |
| Oberseite des nicht gewalzten Bereichs, in Walzrichtung | Oberseite des gewalzten Bereichs, in Walzrichtung | 4,067<br>4,068<br>4,086<br>4,104 | 18<br>18<br>18<br>18 | Nein |
| Oberseite des nicht gewalzten Bereichs, in Walzrichtung | Rückseite des gewalzten Bereichs, quer zur Walzrichtung, äusserster Randbereich | 3,449<br>4,634<br>5,016<br>5,088 | 21<br>23<br>23<br>23 | Nein |

(fortgesetzt)

| Messort des 1. Diffraktogramms | Messort des 2. Diffraktogramms | Chi-Quadrat nach Formel (6) (vier Berechnungen mit verschieden gewähltem Untergrund) | Freiheitsgrade (= Anzahl berück-sichtigte Zählin-tervalle des zusammenhängenden $2\theta$-Bereichs) | Sind die Piken bei 64,8° aus 1. und 2. Diffraktogramm bei einer Signifikanzschranke von 0,001% "hinsichtlich Lage oder Form statistisch signifikant voneinander verschieden"? Ja / Nein |
|---|---|---|---|---|
| Oberseite des nicht gewalzten Bereichs, in Walzrichtung | Rückseite des gewalzten Bereichs, quer zur Walzrichtung, innerer Randbereich | 6,585<br>6,600<br>6,608<br>6,577 | 25<br>25<br>25<br>25 | Nein |
| Oberseite des nicht gewalzten Bereichs, in Walzrichtung | Rückseite des gewalzten Bereichs, quer zur Walzrichtung, Mitte des Streifens | 15,719<br>15,731<br>15,713<br>15,713 | 27<br>27<br>27<br>27 | Nein |
| Oberseite des nicht gewalzten Bereichs, in Walzrichtung | Oberseite des gewalzten Bereichs, quer zur Walzrichtung, äusserster Randbereich | 6,162<br>5,972<br>6,087<br>6,490 | 23<br>23<br>23<br>23 | Nein |
| Oberseite des nicht gewalzten Bereichs, in Walzrichtung | Oberseite des gewalzten Bereichs, quer zur Walzrichtung, innerer Randbereich | 11,851<br>11,991<br>12,010<br>11,922 | 28<br>27<br>27<br>27 | Nein |
| Oberseite des nicht gewalzten Bereichs, in Walzrichtung | Oberseite des gewalzten Bereichs, quer zur Walzrich-tung, Mitte des Streifens | 11,851<br>11,991<br>12,010<br>11,922 | 27<br>27<br>27<br>27 | Nein |

Tabelle 2b (Streifen B)

| Messort des 1. Diffraktogramms | Messort des 2. Diffraktogramms | Chi-Quadrat nach Formel (6) (vier Berechnungen mit verschieden gewähltem Untergrund) | Freiheitsgrade (= Anzahl berück-sichtigte Zählin-tervalle des zusammenhängenden 2θ-Bereichs) | Sind die Piken bei 64,8° aus 1. und 2. Diffraktogramm bei einer Signifikanzschranke von 0,001% "hinsichtlich Lage oder Form statistisch signifikant voneinander verschieden"? Ja / Nein |
|---|---|---|---|---|
| Oberseite des gewalzten Bereichs, quer zur Walzrichtung, Mitte des Streifens | Oberseite des gewalzten Bereichs, quer zur Walzrichtung, Mitte des Streifens | 0,082<br>0,011<br>0,001<br>0,009 | 23<br>22<br>22<br>22 | Nein |
| Oberseite des gewalzten Bereichs, quer zur Walzrichtung, Mitte des Streifens | Oberseite des gewalzten Bereichs, quer zur Walzrichtung, äusserster Randbereich | 4,029<br>4,239<br>3,989<br>4,188 | 24<br>24<br>24<br>24 | Nein |
| Oberseite des gewalzten Bereichs, quer zur Walzrichtung, Mitte des Streifens | Oberseite des gewalzten Bereichs, quer zur Walzrichtung, innerer Randbereich | 32,740<br>33,113<br>33,228<br>33,294 | 26<br>26<br>26<br>26 | Nein |
| Oberseite des gewalzten Bereichs, quer zur Walzrichtung, Mitte des Streifens | Oberseite des gewalzten Bereichs, in Walzrichtung | 12,427<br>12,600<br>12,414<br>12,413 | 22<br>22<br>22<br>22 | Nein |
| Oberseite des nicht gewalzten Bereichs, in Walzrichtung | Oberseite des gewalzten Bereichs, quer zur Walzrichtung, äusserster Randbereich | **122,494**<br>**122,327**<br>**122,538**<br>**123,103** | 24<br>24<br>24<br>24 | **Ja** |
| Oberseite des nicht gewalzten Bereichs, in Walzrichtung | Oberseite des gewalzten Bereichs, quer zur Walzrichtung, innerer Randbereich | **112,289**<br>**112,319**<br>**112,514**<br>**114,382** | 26<br>26<br>26<br>27 | **Ja** |
| Oberseite des nicht gewalzten Bereichs, in Walzrichtung | Oberseite des gewalzten Bereichs, quer zur Walzrichtung, Mitte des Streifens | **138,629**<br>**137,743**<br>**138,966**<br>**138,993** | 24<br>23<br>24<br>24 | **Ja** |

(fortgesetzt)

| Messort des 1. Diffraktogramms | Messort des 2. Diffraktogramms | Chi-Quadrat nach Formel (6) (vier Berechnungen mit verschieden gewähltem Untergrund) | Freiheitsgrade (= Anzahl berück-sichtigte Zählin-tervalle des zusammenhängenden 2θ-Bereichs) | Sind die Piken bei 64,8° aus 1. und 2. Diffraktogramm bei einer Signifikanzschranke von 0,001% "hinsichtlich Lage oder Form statistisch signifikant voneinander verschieden"? Ja / Nein |
|---|---|---|---|---|
| Oberseite des nicht gewalzten Bereichs, in Walzrichtung | Oberseite des gewalzten Bereichs, in Walzrichtung | **81,006** **80,696** **87,205** **87,221** | 19 19 20 20 | **Ja** |

Es ist ersichtlich, dass der herkömmlich gewalzte Streifen B "nach Lage oder Form statistisch signifikant unterschiedliche" Piken bei 64,8° zeigt, wenn Diffraktogramme des nicht gewalzten und des gewalzten Bereichs miteinander verglichen werden. Beim erfindungsgemäss gewalzten Streifen A dagegen sind keine solchen statistisch signifikanten Unterschiede feststellbar, unabhängig von den Messorten der beiden verglichenen Diffraktogramme. Derselbe Befund wird auch dann ermittelt, wenn eine beliebige andere Signifikanzschranke aus Tabelle 1 gewählt wird.

Bevorzugt weist im erfindungsgemässen Streifen der Bereich mit vergleichsweise stärker anisotroper Kristallitorientierung auch eine gegenüber dem Bereich mit vergleichsweise weniger stark anisotroper Kristallitorientierung eine grössere Inhomogenität des Gefüges auf, was ebenfalls durch das erfindungsgemässe Walzverfahren bewirkt wird. Diese Unterschiede in den Gefügehomogenitäten können direkt durch Vergleich von Mikrophotographien an Schnitten des Streifenmaterials aus den fraglichen Gebieten festgestellt werden.

Gleichzeitig bewirkt das erfindungsgemässe Walzverfahren keine inneren Spannungen im Material, was daran erkennbar ist, dass der oder die gewalzten Bereiche bei nachfolgenden Bearbeitungsschritten keine Tendenz zum Verziehen zeigen. Wie jeder Körper weist dieser Metallstreifen drei Hauptträgheitsachsen auf. Da der erfindungsgemässe Metallstreifen eher länglich ist, ist das zu einer der drei Hauptträgheitsachsen zugehörige Trägheitsmoment kleiner als die zu den beiden anderen Hauptträgheitsachsen zugehörigen Hauptträgheitsmomente. Bevorzugt ist dieses kleinste Hauptträgheitsmoment mindestens 10 mal kleiner als die beiden Hauptträgheitsmomente, eher bevorzugt mindestens 50 mal kleiner.

Es hat sich auch gezeigt, dass mittels des erfindungsgemässen Verfahrens gewalzten Bereiche eines Metallstreifens um einen Faktor von bis zu 6 besser federnd sind als herkömmlich gewalzte Bereiche. Streifen, die mittels des erfindungsgemässen Walzverfahrens auf eine bestimmte, konstante Dicke gewalzt wurden, weisen in dem gewalzten Bereich im Vergleich zu einem aus demselben Metall bestehenden Streifen, der aber teilweise mittels eines herkömmlichen Verfahrens mit zwei Walzen auf dieselbe Dicke gewalzt wurde, eine flachere Federkennlinie auf, d.h. es braucht beim erfindungsgemäss gewalzten Streifen zum Erhalt einer bestimmten Biegung weniger Kraft als beim herkömmlich gewalzten, gleich dicken Streifen. Des Weiteren wird die Federkennlinie beim nach erfindungsgemässen Verfahren auf konstante Dicke gewalzten Streifen leicht degressiv, d.h. es braucht mit zunehmender Biegung weniger Kraft, um eine zusätzliche Biegung zu erzeugen. Wenn man beispielsweise die vorstehend beschriebenen Streifen A und B an ihrem 1,5 mm dicken Ende fest einspannt, so beobachtet man die folgenden Auslenkungen in Abhängigkeit bei verschiedenen an dem 0,85 mm dicken, freien Ende im Abstand von 70 mm angehängten Gewichten (Mittelwert aus jeweils 5 Streifen):

Tabelle 3

| Angehängtes Gewicht (g) | Auslenkung (Streifen A) | Auslenkung (Streifen B) |
|---|---|---|
| 50 | 0,07 | 0,05 |
| 100 | 0,14 | 0,10 |
| 150 | 0,21 | 0,15 |
| 200 | 0,29 | 0,20 |

(fortgesetzt)

| Angehängtes Gewicht (g) | Auslenkung (Streifen A) | Auslenkung (Streifen B) |
|---|---|---|
| 250 | 0,38 | 0,25 |

Der Unterschied in den Federkennlinien von erfindungsgemäss gewalztem Streifen A und herkömmlich gewalztem Streifen B wird umso ausgeprägter, je dünner der Streifen ausgewalzt wird, d.h. je kleiner der Stauchgrad (Verhältnis zwischen Streifenhöhe nach dem Walzen und Streifenhöhe vor dem Walzen) ist. Es ist möglich, dass diese Änderung der Federkennlinie mit einer Zunahme der vorstehend erwähnten Inhomogenität des Gefüges zusammenhängt, die bei dem erfindungsgemässen Walzverfahren gegenüber dem nicht gewalzten Bereich des Streifens auftritt. Andererseits ist es mit dem erfindungsgemässen Verfahren möglich, ein Blattfederteil mit progressiver Federkennlinie zu erzielen, indem der zu walzende Bereich auf eine variable Dicke gewalzt wird.

Wenn die vorstehend beschriebenen Streifen A und B an ihren dickeren Enden mit 1,5 mm Walzdicke und an ihren dünneren Enden mit 0,85 mm Walzdicke senkrecht zur Walzrichtung durchgeschnitten werden und diese je zwei Schnittflächen pro Streifen mittels eines Mikrohärtemessgeräts Leitz Miniload 2 gemäss ISO 4516 und ISO 6507/1 auf ihre Vickers-Härte untersucht werden, stellt man die folgenden Werte für die Vickers-Härte (in MPa) fest:

Tabelle 4

| Messung Nr. (in Klammern der Abstand der Eindrückstelle des Stempels vom Streifenrand in mm) | Streifen B (linke Spalte 1,5 mm, rechte Spalte 0,85 mm) | | Streifen A linke Spalte 1,5 mm, rechte Spalte 0,85 mm) | |
|---|---|---|---|---|
| 1 (1,3) | 182 | 296 | 217 | 310 |
| 2 (2,6) | 181 | 302 | 209 | 308 |
| 3 (3,9) | 180 | 301 | 210 | 307 |
| 4 (5,2) | 189 | 302 | 211 | 308 |
| 5 (6,5) | 192 | 306 | 208 | 305 |

Ein vergleichsweise dick erfindungsgemäss gewalzter Bereich zeichnet sich also gegenüber einem auf gleiche Dicke herkömmlich gewalzten Bereich durch eine deutlich höhere Vickers-Härte aus. Der Unterschied wird geringer, wenn dünner ausgewalzt wird.

Ebenfalls feststellbar ist an den gewalzten Stellen, sofern der Metallstreifen aus einer Stahllegierung besteht, in der Regel ein merklicher Anteil von Verformungsmartensit, der typisch im Bereich von etwa 5 bis etwa 30 Volumenprozenten des Metalls liegt.

In einer ersten bevorzugten Variante ist der erfindungsgemässe Metallstreifen in etwa gerade und ist auf mindestens einem Teil seiner Länge mittels des erfindungsgemässen Verfahrens gewalzt. Als "Länge" wird hier die Projektion des Metallstreifens auf seine besagte Hauptträgheitsachse mit kleinstem Trägheitsmoment verstanden.

In einer anderen bevorzugten Ausführungsform ist der mindestens teilweise gewalzte Metallstreifen U-förmig umgebogen, dergestalt, dass er zwei Schenkel aufweist. Jeder dieser Schenkel weist einen oder mehrere (bevorzugt genau einen) Bereich auf, der an die U-förmige Umbiegungsstelle angrenzt, der mittels des erfindungsgemässen Walzverfahrens erhältlich ist, und der die vorstehend erwähnten Eigenschaften aufweist. Die Länge dieses bevorzugten U-förmig umgebogenen Metallstreifens in Projektion auf die vorstehend erwähnte Hauptträgheitsachse mit kleinstem Trägheitsmoment gesehen beträgt bevorzugt etwa 90 bis etwa 200 mm, eher bevorzugt etwa 100 bis etwa 160 mm. Die Länge der nach dem erfindungsgemässen Verfahren gewalzten Bereiche der beiden Schenkel beträgt, in Projektion auf diese Hauptträgheitsachse gesehen, bevorzugt etwa 30 bis etwa 90 mm, eher bevorzugt etwa 40 bis 80 mm. Die Dicke der beiden Schenkel des U-förmig umgebogenen Metallstreifens vor dem Walzen liegt bevorzugt im Bereich von etwa 1 bis etwa 3 mm, bevorzugt im Bereich von etwa 1,25 bis etwa 2,75 mm. Die Dicke der nach dem erfindungsgemässen Verfahren gewalzten Bereiche der Schenkel ist bevorzugt im Bereich von etwa 0,5 bis etwa 1 mm, eher bevorzugt im Bereich von etwa 0,7 bis etwa 0,9 mm. Der Umformungsgrad $\varphi$, berechnet nach der Formel

$$\varphi = \ln\left(\frac{l_1}{l_0}\right) \times 100\% \quad (7)$$

worin $l_1$ die Dicke des gewalzten Bereichs des Schenkels und $l_0$ die Dicke desselben Bereichs vor dem Walzen ist, liegt bevorzugt in einem Bereich von etwa 50% bis etwa 120%. Der U-förmig umgebogene Metallstreifen kann entweder zuerst U-förmig umgebogen werden und dann können an den beiden Schenkeln je ein Bereich mittels eines erfindungsgemässen Walzverfahrens mit zwei Walzen und einer dazwischen liegenden Unterlage gleichzeitig gewalzt werden. Es können auch zunächst an einem noch nicht U-förmig umgebogenen Ausgangsformkörper zwei Bereiche mittels eines erfindungsgemässen Verfahrens mit nur einer Walze und einer Unterlage einzeln gewalzt werden und der Ausgangsformkörper anschliessend zwischen den beiden gewalzten Bereichen U-förmig umgebogen werden. Die Unterlage, auf der gewalzt werden soll, weist in dem Fall bevorzugt eine Oberflächenkontur auf, die genau der Innenkontur des bereits U-förmig umgebogenen Ausgangsformkörpers einschliesslich der beiden zu walzenden Bereiche der beiden Schenkel entspricht. Der zu walzende Ausgangsformkörper kann dann passgenau auf die Unterlage gelegt werden, so dass die beiden zu walzenden Schenkel an beiden Oberflächenseiten der Unterlage herunterhängen. Werden nun die beiden Schenkel gleichzeitig gewalzt, bevorzugt mit einer erfindungsgemässen Walzvorrichtung, die zwei gleiche Teilvorrichtungen mit je einer Walze aufweist; und bevorzugt so, dass die Walzrichtung von oben nach unten ist, so ist ein Verrutschen des Ausgangsformkörpers während des Walzens ausgeschlossen.

Der erfindungsgemässe Metallstreifen eignet sich als Zwischenprodukt zur Herstellung diverser Gegenstände wie vorstehend exemplifiziert. Hierzu kann der Metallstreifen mittels weiterer Verarbeitungsschritte, wie etwa dem Stanzen, Bohren, Fräsen, Biegen, Hobeln oder auch mittels des erfindungsgemässen Walzverfahrens zu einem gewünschten Endprodukt weiterverarbeitet werden.

Wenn der erfindungsgemässe Metallstreifen etwa gerade ist, kann er etwa zu Federn, insbesondere Blattfedern, Spiralfedern oder Uhrfedern, oder Messerklingen weiterverarbeitet werden. In letzterem Fall ist die Klinge des Messers derjenige Teil, der mittels des erfindungsgemässen Verfahrens gewalzt wurde.

Wenn der erfindungsgemässe Metallstreifen, wie erfindungsgemäss bevorzugt, U-förmig umgebogen ist, so kann er zu aus einem Stück bestehenden Instrumenten mit Greiffunktion, wie etwa Pinzetten, Pinzettenscheren, Zangen (z.B. Zuckeroder Eiswürfelzangen) weiterverarbeitet werden. Pinzettenscheren sind Pinzetten, bei denen die beiden freien Enden der Schenkel zu Scherenklingen ausgebildet werden, die beim Zusammendrücken der Schenkel scherend aneinanderreiben. Diese Scherenwirkung kann nach vorne gerichtet oder nach hinten gerichtet sein. Eine Pinzettenschere mit nach hinten gerichteter Scherenwirkung kann durch Ausbilden von Scherenklingen an den Enden der Schenkel und anschliessendem Umbiegen der Enden der Schenkel nach innen und nach hinten erhalten werden.

Für eine Pinzette mit nach vorne gerichteter Scherenwirkung kann im Rahmen der vorliegenden Anmeldung auch der Begriff "vorwärtsschneidende Pinzettenschere" verwendet werden. Für eine Pinzette mit nach hinten gerichteter Scherenwirkung kann im Rahmen der vorliegenden Anmeldung auch der Begriff "rückwärts schneidende Pinzettenschere" verwendet werden.

Die erfindungsgemässen Pinzetten mit oder ohne Scherwirkung lassen sich aufgrund der U-förmigen Biegestelle leichter sterilisieren und reinigen, da an ihrem rückwärtigen Ende keine Fügestelle vorhanden ist, an der die beiden Schenkel in spitzem Winkel zusammenkommen. An dieser bei den vorbekannten Instrumenten vorhandenen spitzwinkligen, nur schlecht zugänglichen Fügestelle können sich Schmutz und Bakterien ansammeln. Die Pinzetten mit oder ohne Scherenwirkung der vorliegenden Erfindung weisen längere und besser federnde Schenkel auf als die vorbekannten Instrumente und erlaubten daher eine feinere Regulierung des Pressdrucks beim manuellen Zusammendrücken oder Loslassen der beiden Schenkel. Die bei vorbekannten Instrumenten vorhandene Schweissstelle am rückwärtigen Ende stellt eine auf Korrosion anfällige Stelle dar, die durch das erfindungsgemässe Walzen und U-förmige Biegen in einem Arbeitsgang (wenn eine Verfahrensvariante mit zwei Teilvorrichtungen mit je einer Walze gewählt wird) ohne Schweissen bei den erfindungsgemässen Pinzetten vermieden wird. Die Enden der beiden Schenkel passen genauer aufeinander, so dass die Nachbearbeitung der Schenkel von Hand, wie sie bei dem vorbekannten Herstellungsverfahren mit Zusammenschweissen der beiden Schenkel oft nötig wird, vermieden wird.

Der erfindungsgemässe Metallstreifen lässt sich auch zur Herstellung von Unterstützungsimplantaten und Gelenkprothesen, die eine beeinträchtigte Gelenkfunktion unterstützen sollen, verwenden. Diese fördern die Distraktion der zusammenwirkenden Teile des Gelenks (Unterstützungsimplantate, insbesondere etwa zur Unterstützung eines Hüft-, Knie- oder anderen Gelenks) oder ersetzen eine verlorene Gelenkfunktion (Gelenkprothesen).

Gemeinsames Merkmal aller solchen Unterstützungsimplantate oder Prothesen ist, dass Blattfederteile, die mittels des erfindungsgemässen Verfahrens gewalzt wurden, in der Hauptbelastungsrichtung des fraglichen Gelenks für Mobilität sorgen. Je nach Bewegungsart des Gelenks, also Flexion/Extrusion, Abduktion/Adduktion, Lateralflexion oder Innen- und Aussenrotation, kommen ein oder mehrere solche Blattfederteile vor, welche vorzugsweise auf Zug belastet werden, aber je nach Konstruktion auch Druck und Torsion aufnehmen können.

Erfindungsgemässe Gelenkprothesen dienen dem Totalersatz eines Gelenks und können grundsätzlich an jedes Gelenk angepasst werden, z.B. die Hüfte, Wirbelsäule, Hand- und Fussgelenke oder das Kiefergelenk. Letzteres ist ein bevorzugtes Beispiel eines Gelenks. Die Befestigung kann entweder bei beiden von dem Gelenk übriggebliebenen Knochenenden an der Beugeseite, bei beiden Knochenenden an der Streckseite oder gekreuzt beim einen Knochenende an der Beugeseite und beim anderen Knochenende an der Streckseite erfolgen. Im Falle der Wirbelsäule kann die Befestigung beidseits in der Gegend der Dornfortsätze / Rippenansätze erfolgen.

Erfindungsgemässe Unterstützungsimplantate umfassen neben den besagten Blattfederteilen U-förmige Biegestellen, die nicht gewalzt sind und in denen die Kristallite daher wiederum vergleichsweise weniger stark anisotrop orientiert sind. Eine "U-förmige" Biegestelle heisst im Rahmen der Erfindung nicht zwingend, dass die Biegestelle eine Umlenkung um 180° bewirkt; "U-förmig" heisst typisch eine Umlenkung um 90° bis 220°, bevorzugt um 160° bis 210°, eher bevorzugt um 170° bis 200°, am meisten bevorzugt um 175° bis 186° oder um genau 180°. Die Blattfederteile selber können plan sein oder selber eine gewisse Krümmung von konstantem oder variablem Radius oder eine Bombierung aufweisen. Die Blattfederteile können auch als progressiv oder partiell wirkender Frosch asugebildet werden, um ihre Streckwirkung zu stabilisieren. Bei den erfindungsgemässen Unterstützungsimplantaten wechseln sich Blattfederteile und Biegungen bevorzugt ab. Bevorzugte Beispiele von Gelenken, die mittels der erfindungsgemässen Unterstützungsimplantate unterstützt werden können, sind etwa Ellipsoidgelenke (Articulatio ellipsoidea); Scharniergelenke (Ginglymus, z. B. die Fingergelenke), Zapfengelenke (Articulatio trochoidea, z.B. das Gelenk zwischen Elle und Speiche); oder bicondyläre Gelenke (Articulatio bicondylaris, etwa das Kniegelenk). Ein besonders bevorzugtes Beispiel sind Kniegelenke.

Die Erfindung wird nun unter Beizug der Figuren weiter veranschaulicht, in welchen Figuren

- Figuren 1 und 2 schematisch zwei Varianten des erfindungsgemässen Walzverfahrens und der zugehörigen Vorrichtung zeigen;

- Figuren 3 bis 6 Pinzetten und Pinzettenscheren zeigen, die unter Verwendung des erfindungsgemäss bevorzugten U-förmig umgebogenen Metallstreifens als Zwischenprodukt erhältlich sind;

- Figuren 7 und 8 ein erfindungsgemässes Unterstützungsimplantat für ein Kniegelenk in gestreckter Stellung des Knies zeigen;

- Figur 9 das Unterstützungsimplantat von Figuren 7 und 8 in Beugestellung des Knies zeigen; und

- Figuren 10 und 11 schematisch die Funktion einer erfindungsgemässen Kiefergelenkprothese zeigen.

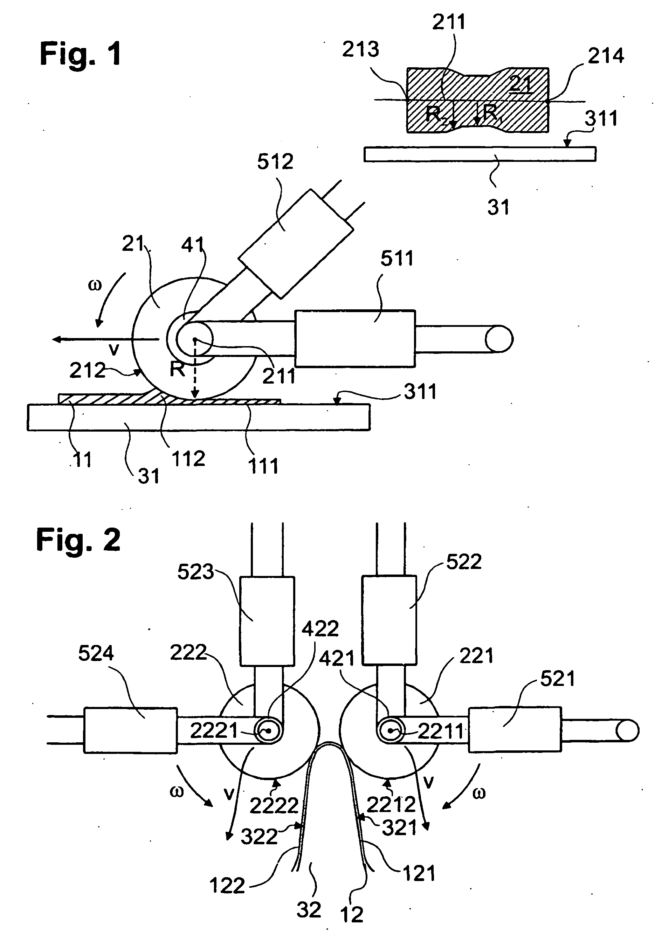

In einer ersten bevorzugten Ausführungsform (Figur 1) zeichnet sich das erfindungsgemässe Verfahren durch nur gerade eine einzige, zylindrische Walze 21 und eine Unterlage 31 aus. Der Ausgangsformkörper 11 wird unter Ausbildung eines mindestens teilweise gewalzten Metallstreifens 111 umgeformt. Gezeigt sind in der Figur auch ein Abstand R, die Winkelgeschwindigkeit ω sowie die Walzgeschwindigkeit v, wie sie in Anspruch 1 verwendet werden. Die Drehachse 2111 der Walze ist hier nur als Punkt gezeigt, da sie senkrecht auf der Blattebene steht. Der Einschub von Figur 1 rechts oben zeigt als Schnitt die zugehörige Walze 21 in Form einer rotationssymmetrischen Profilwalze. Gezeigt ist im Einschub ein Abstand $R_1$ von der Drehachse zu einer walzenden Stelle der Walzenoberfläche, der minimal ist im Vergleich zu einem anderen Abstand $R_2$ einer anderen walzenden Stelle der Walzenoberfläche. Gezeigt sind im Einschub auch die gestrichelte Drehachse 211 und ihre beiden Durchstosspunkte 213, 214 durch die Walze 21. Da hier die Unterlage 31 eine plane Unterlagenoberfläche 311 aufweist, resultiert in der Regel eine gerade Walzrichtung v. In der Figur gezeigt ist eine Reibungsbremse 41, etwa eine Scheibenbremse, die die Bremsung der Winkelgeschwindigkeit ω der Walze 21 bewirken kann. Für das erfindungsgemäss Verfahren ist die Bremse 41 optional, für die erfindungsgemässe Vorrichtung ist sie wesentlich. Gezeigt ist auch, wie sich mittels des erfindungsgemässen Walzverfahrens vor der Walze eine Materialwulst 112 aufbaut, die zum Bremsen der Walze 21 beiträgt. Gezeigt sind auch zwei hydraulische Führungen 511 und 512, die zum Anpressen und Vorwärtsstossen der Walze 21 dienen.

In einer zweiten bevorzugten Ausführungsform des erfindungsgemässen Verfahrens (Figur 2) sind genau zwei Walzen 221, 222 vorhanden, etwa von der Art wie sie im Einschub von Figur 1 gezeigt ist, die je von einer Seite der Unterlage 32 her auf den Ausgangsformkörper 12 (hier vorgängig U-förmig umgebogen, mit zwei Schenkeln 121, 122) walzend einwirken. Ein erster Walzvorgang wird am ersten Schenkel 121 mit einer ersten Walze 221 und an einer ersten Unterlagenoberfläche 321 durchgeführt; gleichzeitig wird ein weiterer Walzvorgang am zweiten Schenkel 122 mit der zweiten Walze 222 und einer zweiten Unterlagenoberfläche 322 durchgeführt, die der ersten Unterlagenoberfläche 321 abgewandt ist. Da hier die Unterlagenoberflächen 321, 322 nicht mehr plan sind, resultiert für die beiden Walzen 221, 222 eine Walzrichtung v, die nicht mehr gerade ist, sondern gekrümmt, und die die Oberflächenkrümmung der Unterlagenoberflächen 321, 322 widerspiegelt. Ebenfalls resultiert hier möglicherweise eine Walzgeschwindigkeit v, die nicht nur nicht mehr gerade, sondern auch betragsmässig variabel ist. Gezeigt sind zwei Reibungsbremsen 421 bzw. 422 (etwa Trommelbremsen), die die Bremsung der Walzen 221 bzw. 222 bewirken können. Für das erfindungsgemäss Verfahren sind die Bremsen optional, für die erfindungsgemässe Vorrichtung sind sie wesentlich. Jede der beiden Walzen 221 bzw. 222 wird wiederum je durch ein Paar von hydraulischen Führungen 521, 522 bzw. 523, 524 auf den Ausgangsformkörper 12 gedrückt und vorwärts gestossen. Die Vorrichtung gemäss Figur 2 könnte aus zwei gleichen Teilvorrichtungen bestehen, einer ersten Teilvorrichtung mit der Walze 221, der Bremse 421 und den hydraulischen Führungen 521, 522; und einer zweiten Teilvorrichtung mit Walze 222, Bremse 422 und hydraulischen Führungen 523, 524; wobei diese Teilvorrichtungen konstruktiv identisch sind und synchron miteinander arbeiten.

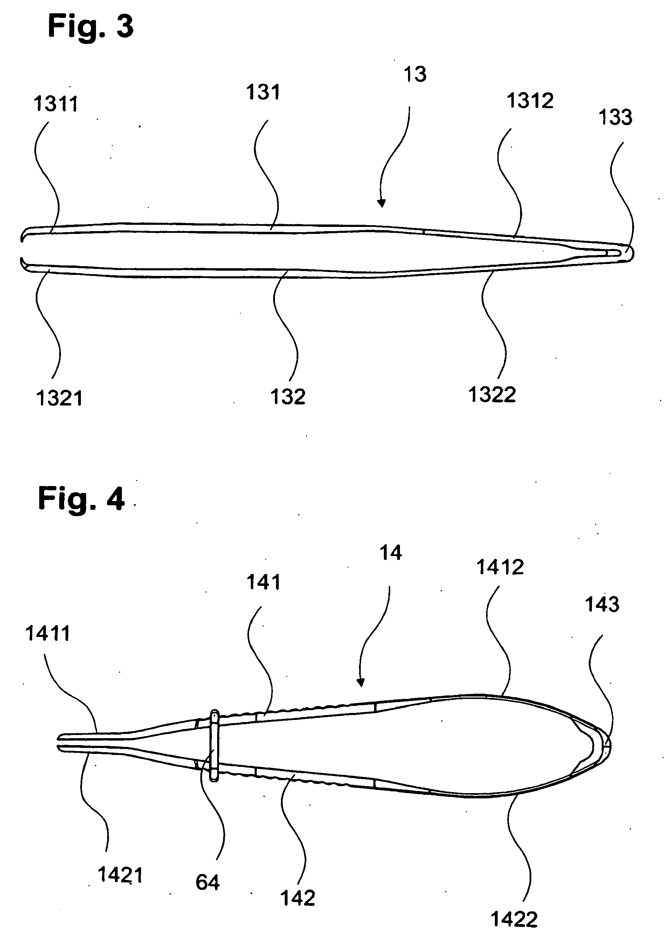

Figuren 3 bis 6 zeigen Ausführungsformen der erfindungsgemässen Pinzetten mit oder ohne Scherenwirkung. Gemeinsame Merkmale aller dieser Pinzetten sind die U-förmige Biegung 133, 143, 153, 163 und die je zwei Schenkel 131/132, 141/142, 151/152 bzw. 161/162, wobei jeder der Schenkel 131 bzw. 132 bzw. 141 bzw. 142 bzw. 151 bzw. 152 bzw. 161 bzw. 162 einen nach dem erfindungsgemässen Verfahren gewalzten Bereich 1312 bzw. 1322 bzw. 1412 bzw. 1422 bzw. 1512 bzw. 1522 bzw. 1612 bzw. 1622 aufweist, der an die Biegung 133 bzw. 143 bzw. 153 bzw. 163 angrenzt. Diese gemeinsamen Merkmale stammen alle aus dem U-förmig umgebogenen Metallstreifen, der als Zwischenprodukt mittels eines Verfahrens und einer Vorrichtung nach Figur 2 erhalten wurde. Diese Pinzetten, ob mit oder ohne Scherenwirkung, bestehen alle bevorzugt aus einer Stahllegierung.

Figur 3 zeigt eine erfindungsgemässe Pinzette 13. Der eine Schenkel 131 weist einen nach dem erfindungsgemässen Verfahren gewalzten Bereich 1312 und der andere Schenkel 132 einen nach dem erfindungsgemässen Verfahren gewalzten Bereich 1322 auf. Die beiden freien Enden 1311, 1321 der beiden Schenkel 131, 132 sind als Spitzen ebenfalls gewalzt und zahnförmig zugestanzt und die Spitzen wurden danach zueinander gekrümmt, so dass die Zähne ineinander greifen können; die hier gezeigte Ausführungsform ist also eine chirurgische Pinzette. Das untere freie Ende 1321 weist einen einzigen Zahn auf, währenddem das obere freie Ende 1311 zwei Zähne aufweist.

Figur 4 zeigt eine erfindungsgemässe Pinzette 14 in Form eines Nadelhalters. Der eine Schenkel 141 weist einen nach dem erfindungsgemässen Verfahren gewalzten Bereich 1412 und der andere Schenkel 142 einen nach dem erfindungsgemässen Verfahren gewalzten Bereich 1422 auf. Die beiden freien Enden 1411, 1421 der beiden Schenkel 141, 142 sind leicht voneinander abgeknickt, um eine Pinzettenspitze mit relativ grosser Auflagefläche zu erhalten, damit eine Nadel festgehalten werden kann. Diese Ausführungsform weist auf den nicht gewalzten Teilen der Schenkel in Querrichtung verlaufende Rillen auf, die in der Figur nur im Profil ersichtlich sind. Diese Querrillen können dazu verwendet werden, um den Nadelhalter 14 mittels eines Halterings 64, der die beiden Schenkel 141, 142 umfasst, in geschlossenem Zustand zu arretieren.

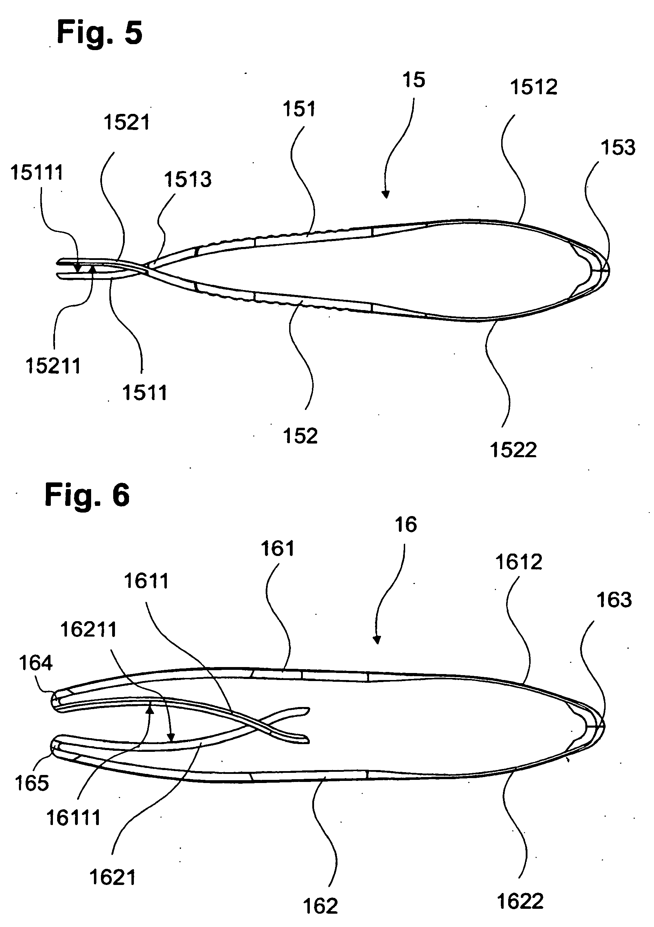

Figur 5 zeigt eine erfindungsgemässe vorwärtsschneidende Pinzettenschere 15. Der eine Schenkel 151 weist mindestens einen nach dem erfindungsgemässen Verfahren gewalzten Bereich 1512 und der andere Schenkel 152 mindestens einen nach dem erfindungsgemässen Verfahren gewalzten Bereich 1522 auf. Die freien Enden 1511 bzw. 1521 überkreuzen sich und weisen je eine Klinge 15111 bzw. 15211 auf. Beim Zusammendrücken der beiden Schenkel 151, 152 wippt der zweite Schenkel 152 über einen im ersten Schenkel 151 ausgebildeten Angelpunkt 1513 (dieser steht in senkrecht zur Blattebene gesehener Richtung etwas nach vorne hervor), wodurch die Enden 1511, 1521 sich aufeinander zubewegen und die Klingen 15111, 15211 vorwärts fortschreitend aneinander scheren.

Figur 6 zeigt eine erfindungsgemässe rückwärtsschneidende Pinzettenschere 16. Der eine Schenkel 161 weist mindestens einen nach dem erfindungsgemässen Verfahren gewalzten Bereich 1612 und der andere Schenkel 162 mindestens einen nach dem erfindungsgemässen Verfahren gewalzten Bereich 1622 auf. Die freien Enden 1611 bzw. 1621 sind über eine erste Knickstelle 164 bzw. eine zweite Knickstelle 165 nach innen und rückwärts in Richtung der U-förmigen Biegestelle 163 umgebogen und weisen je eine Klinge 16111 bzw. 16211 auf, die sich von den besagten Knickstellen 164 bzw. 165 an entlang der gesamten Enden 1611 bzw. 1621 erstreckt. Wenn die Schenkel 161, 162 zusammengedrückt werden, bewegen sich die Knickstellen 164, 165 aufeinander zu und überkreuzen sich, wodurch von da an die Klingen 16111, 16211 sich ebenfalls überkreuzen und von da an nach rückwärts, in Richtung der U-förmigen Biegestelle 163 fortschreitend, scherend aneinander gleiten, so dass eine nach rückwärts wirkende Scherwirkung auftritt.

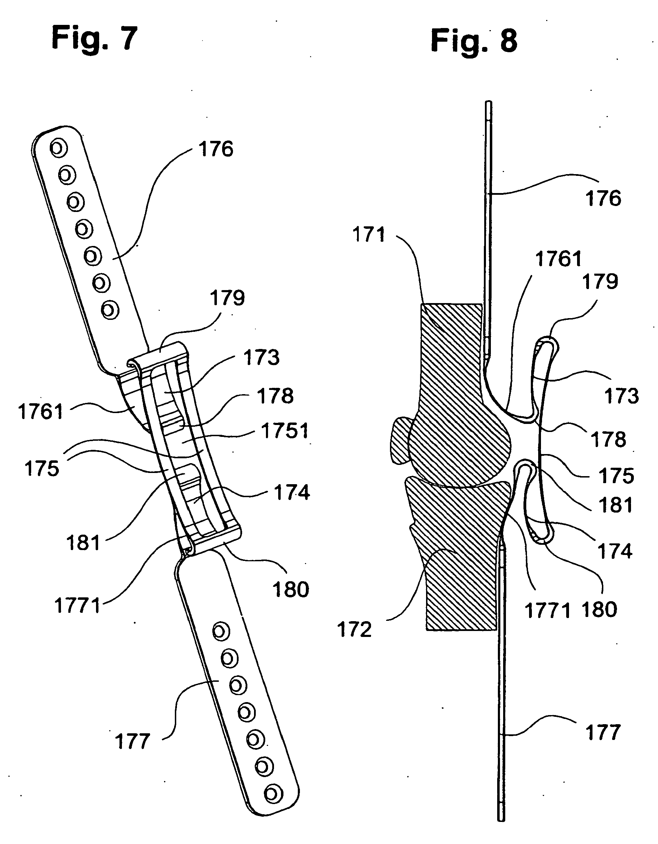

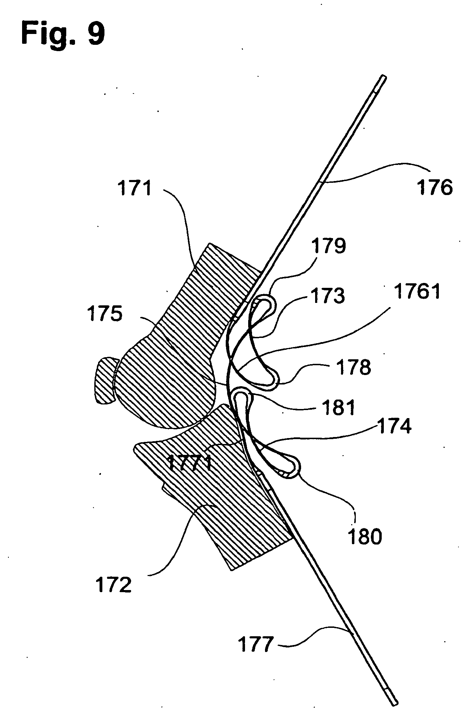

Figuren 7 und 8 zeigen ein erfindungsgemässes Unterstützungsimplantat für ein Kniegelenk. Das Unterstützungsimplantat weist, wenn es von der Seite her betrachtet wird (Figur 8), etwa die Form eines abgeplatteten griechischen Grossbuchstabens Omega "$\Omega$" auf. Der aktive Teil des Unterstützungsimplantates ist in Form einer zusammengesetzten C-Feder, wobei die C-Feder gebildet ist aus drei nach dem erfindungsgemässen Verfahren gewalzten Blattfederteilen 173, 174, 175 und vier U-förmigen Biegungen 178, 179, 180 und 181. Die Blattfederteile sind etwas dünner gezeichnet als die restlichen Teile des aktiven Teils des Unterstützungsimplantates um zu zeigen, dass sie durch den Walzvorgang in der Regel dünner werden. Das Gelenk ist in den Figuren 7 und 8 schematisch durch den Oberschenkelknochen 171 und durch das Wadenbein 172 dargestellt (ebenfalls angedeutet, ohne Bezugszeichen, ist die Kniescheibe). Das Unterstützungsimplantat umfasst des Weiteren zwei Fussteile, die dazu verwendet werden, um das Unterstützungsimplantat an der Beugeseite des Kniegelenks anzubringen. Das erste Fussteil umfasst einen Bereich 176, der in der Regel nicht nach dem erfindungsgemässen Verfahren gewalzt ist und daher in diesem Bereich eine weniger stark anisotrope Kristallitorientierung aufweist als in den drei Blattfederteilen 173, 174, 175, und der in der Regel plattenförmig ist. Das erste Fussteil kann entweder direkt an die erste Biegung 178 angrenzen oder, bevorzugt, über ein drittes kürzeres Blattfederteil 1761, was eine erhöhte Flexibilität ergibt. Analoges gilt für das zweite Fussteil mit seinem nicht nach dem erfindungsgemässen Verfahren gewalzten Bereich 177, das direkt oder, ebenfalls bevorzugt, über ein viertes kürzeres Blattfederteil 1771 an die vierte U-förmige Biegung 181 angrenzen kann. In der in den Figuren 7 und 8 gezeigten Ausführungsform ist das Unterstützungsimplantat zum Befestigen an den Beugeseiten von Oberschenkelknochen 171 und Wadenbein 172 konzipiert; sofern die nicht erfindungsgemäss gewalzten Bereiche 176, 177 anders als wie gezeigt ausgeformt sind, könnte das Unterstützungsimplantat auch beispielsweise an einer anderen Stelle von Oberschenkelknochen 171 und Wadenbein 172 befestigt werden, also auch an der Streckseite oder seitlich am Gelenk. Die drei Blattfederteile 173, 174, 175 sind untereinander und mit den Fussteilen über U-förmig umgebogene, in der Regel nicht erfindungsgemäss gewalzte Biegungen 178, 179, 180, 181 verbunden. Die erste Biegung 178 verbindet das erste Fussteil mit dem ersten kürzeren Blattfederteil 173; die zweite Biegung 179 verbindet das erste kürzere Blattfederteil 173 mit dem längeren Blattfederteil 175; die dritte Biegung 180 verbindet das längere Blattfederteil 175 mit dem zweiten kürzeren Blattfederteil 174 und die vierte Biegung 181 verbindet das zweite kleinere Blattfederteil 174 mit dem zweiten Fussteil. Erstes kürzeres Blattfederteil 173, zweite U-förmige Biegung 179, längeres Blattfederteil 175, dritte U-förmige Biegung 180 und zweites kürzeres Blattfederteil 174 bilden zusammen den besagten aktiven Teil des Unterstützungsimplantates in Form einer C-Feder. Der Rücken dieser C-Feder wird gerade vom längeren Blattfederteil 175 gebildet. Das längere Blattfederteil 175 weist dabei eine Krümmung auf, die den U-förmigen Krümmungen von zweiter U-förmiger Biegung 179 und dritter U-förmiger Biegung 180 entgegengesetzt ist, d.h. konkav ist, und die zur Öffnung der C-Feder hin zeigt. In am Knie befestigtem Zustand weist also die Biegung der C-Feder zum Knie hin, insbesondere zur Kniekehle hin. Das längere Blattfederteil 175 weist eine vorzugsweise langlochförmige oder rechteckige Öffnung 1751 auf, die in Längsrichtung des längeren Blattfederteils 175 verläuft und sich, ebenfalls bevorzugt, über seine gesamte Länge erstreckt. Diese Öffnung 1751 wird beim Beugen des Knies wichtig (siehe Beschrieb von Figur 9 unten). Das Unterstützungsimplantat ist entsprechend der Asymmetrie der Knochenköpfe von Oberschenkelknochen und Wadenbein insgesamt nicht symmetrisch ausgebildet.

Figur 9 zeigt, wie sich das Unterstützungsimplantat von Figuren 7 und 8 zusammenlegt oder zusammenfaltet, wenn das Knie gebeugt ist. Dabei treten erste U-förmige Biegung 178 und vierte U-förmige Biegung 181 durch die Öffnung 1751 im längeren Blattfederteil 175 hindurch, desgleichen mindestens ein Teil des ersten kürzeren Blattfederteils 173 und mindestens ein Teil des zweiten kürzeren Blattfederteils 174. Dazu gegenläufig biegt sich das längere Blattfederteil 175 weiter zur Kniekehle hin durch. Die Aussenkanten der beiden kürzeren Blättfederteile 173, 174 und die längeren Innenkanten der Öffnung 1751 des längere Blattfederteil 175 bewegen sich nahezu scherend aneinander vorbei. Da das Unterstützungsimplantat aufgrund der drei Blattfederteile 173, 174, 175 und der zwei weiteren optionalen Blattfederteile 1761, 1771 über eine hohe Flexibilität in mehreren Richtungen verfügt, könnte es auch eine Protrusionsbewegung oder eine Retrusionsbewegung des Gelenks unter seitlichem Gegeneinanderverschieben von erster Biegung 178 und vierter Biegung 181 nachvollziehen. Durch das Zusammenlegen oder Zusammenfalten weist das Unterstützungsimplantat in gebeugtem Zustand des Knies einen vergleichbar geringen Raumbedarf auf wie in gestrecktem Zustand des Knies.

Das Unterstützungsimplantat der Figuren 7-9 garantiert durch seine Distraktionswirkung in jeder beliebigen Gelenkstellung bei Einwirkung extremer Stauchkräfte die Offenhaltung des Gelenkspaltes, so dass die am Gelenk beteiligten Knochen sich berührungsfrei gegeneinander bewegen können (die durch Abnützung fehlende oder teilweise zerstörte Knorpelschicht ermöglicht das Reiben von Knochen auf Knochen und ist ursächlich für die Schmerzen). Seine von der Seite gesehene Form eines abgeplatteten Omega haben sich nach gegenwärtigem Kenntnisstand der Anmelderin als beste Ausführungsform herausgestellt, um die Bewegungsabläufe eines Kniegelenks möglichst naturnah nachzuvollziehen.

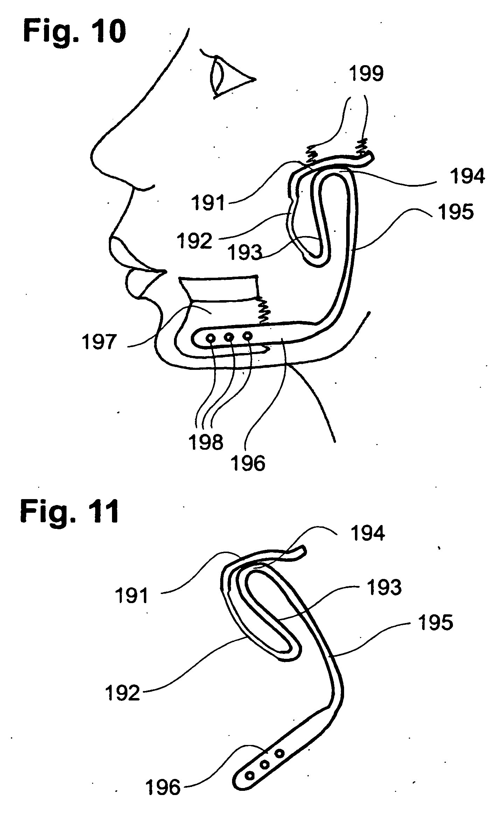

Figuren 10 und 11 zeigen eine erfindungsgemässe Gelenkprothese, die sich insbesondere als Kiefergelenkprothese eignet. Diese umfasst ein Oberkieferteil 191, ein Blattfederteil 192, ein aufsteigendes Schenkelteil 193, ein Stützteil 194, ein absteigendes Schenkelteil 195 und ein Unterkieferteil 196. Figur 10 zeigt die Kiefergelenkprothese in implantiertem Zustand, wenn der Patient den Mund geschlossen hat.

Dabei ist das Oberkieferteil 191 mittels Schrauben 199 an dem verbleibenden Teil des Oberkiefers befestigt und das Unterkieferteil 196 ist mit Schrauben 198 an dem verbleibenden Teil des Unterkiefers befestigt. Das Stützteil 194 liegt von unten an dem Oberkieferteil 191 an; Stützteil 194 und die Unterseite des Oberkieferteils 191 sorgen zusammen für eine das natürliche Kiefergelenk nachahmende Beweglichkeit (Rotation und Verschiebung von Unterkiefer und Oberkiefer gegeneinander). Das Blattfederteil 192, das erfindungsgemäss gewalzt ist, sorgt für die hierzu erforderliche federnde Flexibilität der Prothese. Figur 11 zeigt dieselbe Kiefergelenkprothese nocheinmal, wenn der Patient den Mund geöffnet hat. Dabei biegt sich das Blattfederteil 192 etwas nach rückwärts und streckt sich etwas, und das Stützteil 194 dreht sich zusammen mit absteigendem Schenkelteil 195 und Unterkieferteil 196 nach unten, wobei das Stützteil 194 gleichzeitig an der Unterseite des Oberkieferteils 191 etwas nach vorne gleiten kann. Diese Kiefergelenkprothese verhindert übermässige Distraktionsbewegungen von Ober- und Unterkiefer: Die Verhinderung dieser Distraktion setzt dann ein, wenn das Blattfederteil 192 nahezu völlig gerade ist.

**Patentansprüche**

1. Einstückiger Metallstreifen ohne Schweissnähte und aus einer polykristallinen Stahllegierung, umfassend mindestens einen Bereich, in dem die Kristallite vergleichsweise stärker anisotrop orientiert sind, und mindestens einen Bereich, in dem die Kristallite vergleichsweise weniger stark anisotrop orientiert sind; und wobei mit CuKα-Strahlung an zwei beliebigen Stellen des Streifens gemessene θ-2θ-Röntgendiffraktogramme keine statistisch signifikanten Unterschiede hinsichtlich Lage oder Form zwischen den jeweils entsprechenden Piken zeigen.

2. Metallstreifen nach Anspruch 1, **dadurch gekennzeichnet, dass** die Stahllegierung ein im kubisch innenzentrierten Kristallgitter kristallisierender ferritische, martensitische oder gemischt ferritisch/martensitische Stahl, bevorzugt ein Stahl der Zusammensetzung Cr 12,50-14,50 Gewichtsprozent, C 0,42-0,50 Gewichtsprozent, Si max. 1,00 Gewichtsprozent, Mn max. 1,00 Gewichtsprozent, P max. 0,045 Gewichtsprozent, S max. 0,030 Gewichtsprozent, Rest im Wesentlichem Eisen und unvermeidbare Verunreinigungen, ist.

3. Metallstreifen nach einem der Ansprüche 1 bis 2, **dadurch gekennzeichnet, dass** der Bereich mit vergleichsweise stärker anisotroper Kristallitorientierung auch eine vergleichsweise stärkere Inhomogenität des Gefüges aufweist als der Bereich mit vergleichsweise weniger stark anisotroper Kristallitorientierung.

4. Walzverfahren zur Umformung eines metallischen Ausgangsformkörpers (11, 12) aus eines polykristallinen Stahllegierung, wobei der walzvorgang zwischen einer Walze (21, 221, 222) mit einer Drehachse (211, 2211, 2221) und mit einer Walzenoberfläche (212, 2212, 2222) einerseits und einer Unterlage (31, 32) mit einer Unterlagenoberfläche (311, 321, 322) andererseits durchgerührt wird, **dadurch gekennzeichnet, dass** die Winkelgeschwindigkeit ω der walze so gesteuert wird, dass an mindestens einer Stelle der Walzenoberfläche (212, 2212, 2222), die mit dem Ausgangsformkörper (11, 12) walzend in Berührung kommt,

$$0 \leq \omega < \frac{v}{R}, \quad (1)$$

gilt, in welcher Formel v die Walzgeschwindigkeit ist, mit der sich ein gedachter Punkt, der auf der Drehachse (211, 2211, 2221) der Walze (21, 221, 222) in der Mitte zwischen den Durchstosspunkten der Drehachse (211, 2211, 2221) durch die beiden Stirnflächen der Walze (21, 221, 222) liegt, relativ zum Ausgangsformkörger (11, 12) vor seinem Eintritt in die Walzzone zwischen Walze (21, 221, 222) und Unterlage (31, 32) bewegt; und R der senkrecht zur Drehachse (211, 2211, 2221) der Walze (21, 221, 222) gemessene Abstand zwischen der Drehachse (211, 2211, 2221) und der besagten stelle der, Walzenoberfläche (212, 2212, 2222) ist, und dass die Temperatur des Ausgangeformkörperz und des gewalzten Metallstreifens während des gesamten walzvorgangs an keinem Ort 100°C übersteigt.

5. Verfahren nach Anspruch 4, **dadurch gekennzeichnet, dass** die Winkelgeschwindigkeit ω der Walze so gesteuert wird, dass an jeder Stelle der Walzenoberfläche (212, 2212, 2222), die mit dem Ausgangsformkörper (11, 12) walzend in Berührung kommt,

$$0 \leq \omega < \frac{v}{R}, \quad (1)$$

gilt, wobei ω, v und R dieselbe Bedeutung haben wie im Anspruch 4.

6. Verfahren nach Anspruch 4 oder 5, **dadurch gekennzeichnet, dass** der Ausgangsformkörper (12) U-förmig umgebogen ist, dergestalt, dass er zwei Schenkel (121, 122) aufweist, dass die Unterlage (32) eine erste Unterlagenoberfläche (321) und eine zweite Unterlagenoberfläche (322) aufweist, dass der erste Schenkel (121) zwischen einer ersten Walzenoberfläche (2212) einer ersten Walze (221) und der ersten Unterlagenoberfläche (321) gewalzt wird und gleichzeitig der zweite Schenkel (122) zwischen einer zweiten Walzenoberfläche (2222) einer zweiten Walze (222) und der zweiten Unterlagenoberfläche (322) gewalzt wird.

7. Verfahren nach einem der Ansprüche 4 bis 6, **dadurch gekennzeichnet, dass** die Winkelgeschwindigkeit ω jeder Walze (21, 221, 222) im Bereich von 30 bis 95 %, bevorzugt 50 bis 80 % des Quotienten v/R liegt.

8. Verfahren nach einem der Ansprüche 4 bis 7, **dadurch gekennzeichnet, dass** die Bremsung der Walze oder der Walzen (21, 221, 222) mittels einer Reibungsbremse, etwa einer Scheibenbremse (41) oder einer Trommelbremse (421, 422) oder mittels einer Wirbelstrombremse bewirkt wird, oder dass die Steuerung der Winkelgeschwindigkeit der, Walze über die Drehzahl eines elektrischen oder hydraulischen Motors bewirkt wird.

9. Verfahren nach einem der Ansprüche 4 bis 8, **dadurch gekennzeichnet, dass** der Ausgangsformkörper (11, 12) aus einer ferritischen, martensitischen oder gemischt ferritisch/martensitischen Stahllegierung, eher bevorzugt aus einem Strahl der Zusammensetzung Cr 12,50-14,50 Gewichtsprozent, C 0, 42-0, 50 Gewichtsprozent, Si max. 1,00 Gewichtsprozent, Mn max. 1,00 Gewichtsprozent, P. max. 0,045 Gewichtsprozent, S max. 0,030 Gewichtsprozent, Rest im Wesentlichem Eisen und unvermeidbare Verunreinigungen, besteht.

10. Verfahren nach einem der Ansprüche 4 bis 9, dadurch gekennzeichet, dass die Walze eine Profilwalze ist.

11. Walzvorrichtung zur Durchführung das Verfahrens nach Anspruch 4, umfassend eine Walze (21, 221, 212) mit einer Drehachse (211, 2211, 2221) und einer Walzenoberfläche (212, 2212, 2222), eine Unterlage (31, 32) mit einer Unterlagenoberfläche (311, 321, 322) und eine Bremse (41 bzw. 421 bzw. 422), die während des Walzens zur Bremsung der Walze (21 bzw. 221 bzw. 222) befähigt ist,

12. Vorrichtung nach Anspruch 11, **dadurch gekennzeichnet, dass** sie eine erste Walze (221) mit einer ersten Drehachse (2211) und einer ersten Walzenoberfläche (2212), eine erste Bremse (421), eine zweite Walze (222) mit einer zweiten Drehachse (2221) und einer zweiten Wolzeneberfläche (2222), eine zweite Bremse (422) und eine Unterlage (32) mit einer ersten Unterlagenoberfläche (321) und einer zweiten Unterlagenoberfläche (322) aufweist, dass die erste Walze (221) zur Ausführung eines Walzvorgangs auf der ersten Unterlagenoberfläche (321) befähigt ist und die zweite Walze (222) zur gleichzeitigen Ausführung eines Walzvorgangs auf der zweiten unterlagenoberfläche (322) befähigt ist, und dass die erste Bremse (421) zur Bremsung der ersten Walze (221) und die zweite Bremsen (422) zur Bremsung der zweiten Walze (222) befähigt ist.

13. Aus einem Metallstreifen nach Anspruch 1 bestehende Pinzette (13, 14, 15, 16) mit einer U-förmigen Biegestelle (133, 143, 153, 163) und zwei federnden Schenkeln (131/132, 141/142, 151/152, 161/162), wobei jeder Schenkel (131 bzw. 132 bzw. 141 bzw. 142 bzw. 151 bzw. 152 bzw. 161 bzw. 162) ein distales Ende (1311 bzw. 1321 bzw. 1411 bzw. 1421 bzw. 1511 bzw. 1521 bzw. 1611 bzw. 1621) aufweist, **dadurch gekennzeichnet, dass** die Schenkel (131 bzw. 132 bzw. 141 bzw. 141 bzw. 151 bzw. 152 bzw. 161 bzw. 162) mindestens je einen Bereich (1312 bzw. 1322 bzw. 1412 bzw. 1422 bzw. 1512 bzw. 1522 bzw. 1612 bzw. 1622) aufweisen, der nach dem Walzverfahren gemäss einem der Anspruche 4 bis 10 erhältlich ist, oder in dem die Kristallitorientierung vergleichsweise stärker anisotrop ist, die Biegestelle der Pinzette (133, 143, 153, 163) ein Bereich ist, in dem die Kristallitorientierung vergleichsweise weniger stark anisotrop ist, und mit CuKα-Strahlung an zwei beliebigen Stellen der Pinzette gemessene θ-2θ-Röntgendiffraktogramme keine statistisch signifikanten Unterschiede hinsichtlich Lage oder Form der jeweils entsprechenden Piken zeigen.

14. Pinzette (15) nach Anspruch 13, **dadurch gekennzeichnet, dass** die distalen Enden (1511 bzw. 1521) eine Klinge (15111 bzw. 15211) aufweisen und dass der erste Schenkal (151) einen Angelpunkt (1513) aufweist, dergestalt, dass beim Zusammendrücken der Schankel (151, 152) der zweite Schenkel (152). zum Wippen über den Angelpunkt (1513) bafähigt ist, die Enden (1511, 1521) zum Aufeinanderzubewegen befähigt sind, und die Klingen (15111, 15211) während dieses Aufeinanderzubewegens zum scherenden Anseinandergleiten befähigt sind.

15. Pinzette nach einem der Ansprüche 13 bis 14, wobei die Stahllegierung ein ferritische marrensitische oder gemischt ferritisch/martensititischer Stahl, eher bevorzugt ein Stahl der Zusammensetzung Cr 12.50-14, 50 Gewichtsprozent, C 0, 42-0, 50 Gewichtsprozent, Si max. 1,00 Gewichtsprozent, Mn max. 1,00 Gewichtsprozent, P max. 0,045 Gewichtsprozent, S max. 0,030 Gewichtsprozent und Rest im Wesentlichen Eisen und unvermeidbare Verunreinigungen ist.

16. Unterstützungsimplantat für ein Gelenk, das ein distales und ein proximales Knochenende umfasst, wobei diese Knochenenden gelenkig miteinander verbunden sind, und wobei das Unterstützungsimplantat einen Metallstreifen nach einem der Ansprüche 1 bis 3 umfasst oder aus diesen besteht.

**17.** Gelenkprothese zum Totalersatz eines Gelenks, umfassend einen oder bestehend aus einem Metallstreifen nach einem der Ansprüche 1 bis 3.

**18.** Gelenkprothese nach Anspruch 17 zum Totalerests eines Kiefergelenks, wobei der Metallestreifen einen Bereich in Form eines Blattfederteils (192) aufweist, der eine vergleichsweise stärker anisotrope Kristallitorientierung aufweist oder mittels das Walsververfahrens nach einen der Ansprüche 4 bis 10 erhältlich ist, und fünf Bereiche mit bevorzugt vergleichsweise weniger stark anisotroper Kristallitorientierung in Form eines Oberkisferteile (191), eines aufsteigenden Schankelteils (193), eines Stützteils (194), eines absteigenden Schenkelteils (195) und eines Unterkieferteils (196) aufweist; dergestalt, dass an das Oberkieferteil (191) das Blattfederteil (192) angrenzt, an das Blattfederteil (192) das aufsteigende Schenkelteil (193) angrenzt, an das aufsteigende Schenkelteil (193) das Stützteil (194) angrenzt, an das Stützteil (194) das absteigende Schenkelteil (195) angrenzt und an das absteigende Schenkelteil (195) das Unterkieferteil (196) angrenzt, und dergestalt, dass Blattfederteil (192) und aufstsigendes Schenkelteil (193) eine erste Schlaufe bilden, daren Öffhung zum Oberkieferteil (191) hin zeigt, und aufsteigendes Schenkelteil (193), Stützteil (194) und absteigendes Schenkelteil (195) eine zweite Schlaufe bilden, deren Öffnung in umgekehrter Richtung zeigt, Stützteil (194) und Oberkieferteil (191) sich dergestalt berühren können, dass das Stützteil (194) auf dem Oberkieferteil (191) abrollen und auf ihm entlanggleiten kann, so dass aufgrund von Blattfederteil (192) und der Berührungestelle zwischen Oberkieferteil (191) und Stützteil (194) die Prothese die Bewegungsmöglichkeiten eines Kiefergelenks aufweist.

**Claims**

**1.** A single-piece metal strip having no weld seams and made of a polycrystalline steel alloy, comprising at least one region in which the crystallites have a comparatively more anisotropic orientation, and at least one region in which the crystallites have comparatively less anisotropic orientation; and whereby θ-2θ X-ray diffractograms measured at two arbitrary points of the strip using CuKα radiation show no statistically significant differences with respect to the position or shape between the corresponding pikes.

**2.** The metal strip of claim 1, **characterised in that** the steel alloy is a ferritic, martensitic, or mixed ferritic/martensitic steel crystallising in the body-centred cubic crystal lattice, preferably a steel of the composition Cr 12.50-14.50 percent by weight, C 0.42-0.50 percent by weight, Si max. 1.00 percent by weight, Mn max. 1.00 percent by weight, P max. 0.045 percent by weight, S max. 0.030 percent by weight, the rest being essentially iron and unavoidable contaminants.

**3.** The metal strip of any of claims 1 to 2, **characterised in that** the region in which the crystallite orientation is comparatively more anisotropic also has a microstructure with comparatively more pronounced inhomogeneity than the region in which the crystallite orientation is comparatively less anisotropic.

**4.** A rolling process for shaping a metallic initial shaped body (11, 12) of a polycrystalline steel alloy, wherein the rolling operation wherein the rolling process is performed between a roll (21, 221, 222) having an axis of rotation (211, 2211, 2221) and a rolling surface (212, 2212, 2222) on the one hand, and a support (31, 32) having a support surface (311, 321, 322) on the other hand; **characterized in that** the angular velocity ω of the roll is controlled in such a manner that

$$0 \le \omega < \frac{v}{R} \qquad (1)$$

applies for at least one point of the roll surface (212, 2212, 2222) that contacts the initial shaped body (11, 12) in rolling manner, and in which formula v is the rolling velocity at which an imaginary point on the axis of rotation (211, 2211, 2221) of the roll (21, 221, 222), lying equidistantly between the points where the axis of rotation (221, 2211, 2221) intersects the two frontal faces of the roll (21, 221, 222), moves relative to the initial shaped body (11, 12) before it enters the rolling zone between the roll (21, 221, 222) and the support (31, 332); and R is the distance between the axis of rotation (211, 2211, 2221) and the said point on the roll surface (212, 2212, 2222) measured perpendicularly to the axis of rotation (211, 2211, 2221) of the roll; and **in that** the temperature of the initial shaped body and the rolled metal strip during the entire rolling process does not exceed 100°C at any point.

**5.** The process of claim 4, **characterised in that** the angular velocity ω of the roll is controlled in such manner that

$$0 \le \omega < \frac{v}{R}, \quad (1)$$

applies for each point of the roll surface (212, 2212, 2222) that contacts the initial shaped body (11, 12) in rolling manner, wherein ω, v and R mean the same as in claim 4.

6.   The process of claim 4 or 5, **characterised in that** the initial shaped body (12) is bent in a U-shape such that it has two legs (121, 122), that the support (32) has a first support surface (321) and a second support surface (322), that the first leg (121) is rolled between a first rolling surface (2212) of a first roll (221) and the first support surface (321), and at the same time the second leg (122) is rolled between a second rolling surface (2222) of a second roll (222) and the second support surface (322).

7.   The process of one of claims 4 to 6, **characterised in that** the angular velocity ω of each roll (21, 221, 222) is in the range of 30 to 95%, preferably 50 to 80% of the quotient v/R.

8.   The process of one of claims 4 to 7, **characterised in that** braking of the roll or rolls (21, 221, 222) is effected with a friction brake, for example a disc brake (41) or a drum brake (421, 422) or by means of an eddy current brake, or that the angular velocity of the roll is controlled via the rotating speed on an electric or hydraulic motor.

9.   The process of one of claims 4 to 8, **characterised in that** the initial shaped body (11, 12) consists of a ferritic, martensitic, or mixed ferritic/martensitic steel alloy, more preferably of a steel of a composition Cr 12.50-14.50 percent by weight, C 0.42-0.50 percent by weight, Si max. 1.00 percent by weight, Mn max. 1.00 percent by weight, P max. 0.045 percent by weight, S max. 0.030 percent by weight, the rest being essentially iron and unavoidable contaminants.

10.   The process of one of claims 4 to 9, **characterized in that** the roll is a profile roll.

11.   A rolling device for performing the process of claim 4, comprising a roll (21, 221, 222) with an axis of rotation (211, 2211, 2221) and a rolling surface (212, 2212, 2222), a support (31, 32) with a support surface (311, 321, 322) and a brake (41 or 421 or 422, respectively), which is able to brake the roll (21 or 221 or 222, respectively) during rolling.

12.   The device of claim 11, **characterised in that** it comprises a first roll (221) with a first axis of rotation (2211) and a first rolling surface (2212), a first brake (421), a second roll (222) with a second axis of rotation (2221) and a second rolling surface (2222), a second brake (422) and a support (32) with a first support surface (321) and a second support surface (322); that the first roll (221) is able to perform a rolling action on the first support surface (321) and the second roll (222) is able to perform a rolling action on the second support surface (322) at the same time; and that the first brake (421) is able to brake the first roll (221) and the second brake (422) is able to brake the second roll (222).

13.   A tweezers (13, 14, 15, 16) consisting of a metal strip according to claim 1, with a U-shaped bending point (133, 143, 153, 163) and two resilient legs (131/132, 141/142, 151/152, 161/162), whereby each leg (131, 132, 141, 142, 151, 152, 161 and 162, respectively) has a distal end (1311, 1321, 1411, 1421, 1511, 1521, 1611 and 1621, respectively), **characterised in that** the legs (131, 132, 141, 142, 151, 152, 161 and 162, respectively) each have at least one region (1312, 1322, 1412, 1422, 1512, 1522, 1612 and 1622, respectively), which is obtainable according to the rolling process of one of claims 4 to 10, or in which the crystallite orientation is comparatively more anisotropic; the bending point of the tweezers (133, 143, 153, 163) is a region in which the crystallite orientation is comparatively less anisotropic, and θ-2θ X-ray diffractograms measured at two arbitrary points of the tweezers using CuKα radiation show no statistically significant differences with respect to the position or shape between the corresponding pikes.

14.   The tweezers (15) of claim 13, **characterised in that** the distal ends (1511 or 1521, respectively) have a blade (15111 and 15211, respectively) and that the first leg (151) has a pivot point (1513) designed such that when the legs (151, 152) are squeezed together the second leg (152) is able to rock over the pivot point (1513), the ends (1511, 1521) are able to move towards one another, and the blades (15111, 15211) are able to slide past one another in a shearing manner during this approaching motion.

15.   The tweezers of one of claims 13 to 14, whereby the steel alloy is a ferritic, martensitic or mixed ferritic/martensitic steel, more preferably a steel with the composition Cr 12.50-14.50 percent by weight, C 0.42-0.50 percent by weight, Si max. 1.00 percent by weight, Mn max. 1.00 percent by weight, P max. 0.045 percent by weight, S max. 0.030 percent by weight, the rest being essentially iron and unavoidable contaminants.

16. A supporting implant for a joint, comprising a distal and a proximal bone end, whereby these bone ends are connected to one another in articulated manner, and whereby the supporting implant comprises a metal strip according to one of claims 1 to 3, or consists thereof.

17. A joint prosthesis for complete replacement of a joint, comprising a metal strip or consisting of a metal strip of one of claims 1 to 3.

18. The joint prosthesis of claim 17 for complete replacement of a mandibular joint, whereby the metal strip has a region in the form of a leaf spring part (192) which has comparatively more anisotropic crystallite orientation or which is obtainable using the rolling process of one of claims 4 to 10, and five regions preferably with comparatively less anisotropic crystallite orientation in the form of an upper jaw part (191), an ascending leg part (193), a support part (194), a descending leg part (195) and a lower jaw part (196); such that the leaf spring part (192) adjoins the upper jaw part (191), the ascending leg part (193) adjoins the leaf spring part (192), the support part (194) adjoins the ascending leg part (193), the descending leg part (195) adjoins the support part (194), and the lower jaw part (196) adjoins the descending leg part (195); and such that leaf spring part (192) and ascending leg part (193) form a first loop, the opening of which faces towards the upper jaw part (191), and ascending leg part (193), support part (194) and descending leg part (195) form a second loop, the opening of which faces in the opposite direction; support part (194) and upper jaw part (191) are able to contact one another in such manner that the support part (194) is able to roll over the upper jaw part (191) and slide along it, so that due to the leaf spring part (192) and due to the point of contact between the upper jaw part (191) and the support part (194) the prosthesis has the movement capabilities of a mandibular joint.

## Revendications

1. Ruban métallique en une seule pièce, sans cordon de soudure et en alliage d'acier polycristallin, comprenant au moins une zone où les cristallites sont orientés avec une anisotropie relativement élevée, et au moins une zone où les cristallites sont orientés avec une anisotropie comparativement moins élevée; et où des diffractogrammes θ - 2θ de rayons X mesurés à deux emplacements quelconques du ruban par rayonnement CuKα ne présentent aucune différence statistiquement significative quant à la position ou à la forme des pics correspondants.

2. Ruban métallique selon la revendication 1, **caractérisé en ce que** l'alliage d'acier est un acier ferritique, martensitique ou mixte ferritique/martensitique cristallisant dans le réseau cristallin cubique centré, préférentiellement un acier de composition Cr 12,50-14,50 % en poids, C 0,42-0,50 % en poids, Si max. 1,00 % en poids, Mn max. 1,00 % en poids, P max. 0,045 % en poids, S max. 0,030 % en poids, le reste étant essentiellement du fer et des impuretés inévitables.

3. Ruban métallique selon l'une des revendications 1 ou 2, **caractérisé en ce que** la zone aux cristallites orientés avec une anisotropie relativement élevée présente aussi une inhomogénéité de structure relativement plus élevée que la zone aux cristallites orientés avec une anisotropie comparativement moins élevée.

4. Procédé de laminage pour la déformation d'un corps métallique moulé initial (11, 12) en alliage d'acier polycristallin, où le processus de laminage est exécuté entre d'une part un cylindre (21, 221, 222) comportant un axe de rotation (211, 2211, 2221) et une surface de cylindre (212, 2212, 2222), et d'autre part un support (31, 32) comportant une surface de support (311, 321, 322); **caractérisé en ce que** la vitesse angulaire ω du cylindre est contrôlée de manière qu'à au moins un emplacement de la surface du cylindre (212, 2212, 2222) qui entre en contact de laminage avec le corps moulé initial (11, 12), la formule :

$$0 \leq \omega < \frac{v}{R} \qquad (1)$$

s'applique, dans laquelle v est la vitesse de laminage à laquelle un point théorique sur l'axe de rotation (211, 2211, 2221) du cylindre (21, 221, 222), situé au milieu entre les points d'intersection de l'axe de rotation (211, 2211, 2221) avec les deux surfaces frontales du cylindre (21, 221, 222), se déplace relativement au corps moulé initial (11, 12) avant son entrée dans la zone de laminage entre le cylindre (21, 221, 222) et le support (31, 32) ; et où R est la distance mesurée perpendiculairement à l'axe de rotation (211, 2211, 2221) du cylindre (21, 221, 222) entre l'axe de rotation (211, 2211, 2221) et ledit emplacement de la surface de cylindre (212, 2212, 2222) ; et **en ce que** la température du corps moulé initial et du ruban métallique laminé ne dépasse pas 100°C à aucun emplacement pendant tout le processus de laminage.

5. Procédé selon la revendication 4, **caractérisé en ce que** la vitesse angulaire ω du cylindre est contrôlée de manière qu'à chaque emplacement de la surface de cylindre (212, 2212, 2222) qui entre en contact de laminage avec le corps moulé initial (11, 12), la formule :

$$0 \le \omega < \frac{v}{R} \qquad (1)$$

s'applique, où ω, v et R ont la même signification que dans la revendication 4.

6. Procédé selon la revendication 4 ou la revendication 5, **caractérisé en ce que** le corps moulé initial (12) est plié en U, de manière à présenter deux branches (121, 122), **en ce que** le support (32) présente une première surface de support (321) et une deuxième surface de support (322), **en ce que** la première branche (121) est laminée entre une première surface de cylindre (2212) d'un premier cylindre (221) et la première surface de support (321), et **en ce que** la deuxième branche (122) est simultanément laminée entre une deuxième surface de cylindre (2222) d'un deuxième cylindre (222) et la deuxième surface de support (322).

7. Procédé selon l'une des revendications 4 à 6, **caractérisé en ce que** la vitesse angulaire ω de chaque cylindre (21, 221, 222) est dans la gamme de 30 à 95 %, préférentiellement de 50 à 80 % du quotient v/R.

8. Procédé selon l'une des revendications 4 à 7, **caractérisé en ce que** le freinage du ou des cylindres (21, 221, 222) est effectué au moyen d'un frein à friction, tel qu'un frein à disque (41) ou un frein à tambour (421, 422) ou au moyen d'un frein électromagnétique, ou **en ce que** le contrôle de la vitesse angulaire du cylindre est effectuée par la vitesse de rotation d'un moteur électrique ou hydraulique.

9. Procédé selon l'une des revendications 4 à 8, **caractérisé en ce que** le corps moulé initial (11, 12) est d'un alliage d'acier ferritique, martensitique ou mixte ferritique/martensitique, tout particulièrement un acier de composition Cr 12,50-14,50 % en poids, C 0,42-0,50 % en poids, Si max. 1,00 % en poids, Mn max. 1,00 % en poids, P max. 0,045 % en poids, S max. 0,030 % en poids, le reste étant essentiellement du fer et des impuretés inévitables.

10. Procédé selon l'une des revendications 4 à 9, **caractérisé en ce que** le cylindre est un cylindre profilé.

11. Système de laminage pour l'exécution du procédé selon la revendication 4, comprenant un cylindre (21, 221, 222) avec un axe de rotation (211, 2211, 2221) et une surface de cylindre (212, 2212, 2222), un support (31, 32) avec une surface de support (311, 321, 322) et un frein (41 ou 421 ou 422) apte à freiner le cylindre (21 ou 221 ou 222) pendant le laminage.

12. Système selon la revendication 11, **caractérisé en ce qu'**il comprend un premier cylindre (221) avec un premier axe de rotation (2211) et une première surface de cylindre (2212), un premier frein (421), un deuxième cylindre (222) avec un deuxième axe de rotation (2221) et une deuxième surface de cylindre (2222), un deuxième frein (422) et un substrat (32) avec une première surface de support (321) et une deuxième surface de support (322) ; **en ce que** le premier cylindre (221) est apte à exécuter un processus de laminage sur la première surface de support (321) et le deuxième cylindre (222) est apte à exécuter simultanément un processus de laminage sur la deuxième surface de support (322) ; et **en ce que** le premier frein (421) est apte à freiner le premier cylindre (221) et le deuxième frein (422) est apte à freiner le deuxième cylindre (222).

13. Pince (13, 14, 15, 16) consistant d'un ruban métallique selon la revendication 1, avec un point de pliage en U (133, 143, 153, 163) et deux branches (131/132, 141/142, 151/152, 161/162) élastiques, chaque branche (131 ou 132 ou 141 ou 142 ou 151 ou 152 ou 161 ou 162) présentant une extrémité distale (1311 ou 1321 ou 1411 ou 1421 ou 1511 ou 1521 ou 1611 ou 1621), **caractérisée en ce que** les branches (131 ou 132 ou 141 ou 142 ou 151 ou 152 ou 161 ou 162) comprennent chacune au moins une zone (1312 ou 1322 ou 1412 ou 1422 ou 1512 ou 1522 ou 1612 ou 1622) obtenable par le procédé de laminage selon une des revendications 4 à 10, ou dans laquelle les cristallites sont orientés avec une anisotropie relativement élevée ; **en ce que** le point de pliage de la pince (133, 143, 153, 163) est une zone où les cristallites sont orientés avec une anisotropie comparativement moins élevée, et où des diffractogrammes θ - 2θ de rayons X mesurés à deux emplacements quelconques du ruban par rayonnement CuKα ne présentent aucune différence statistiquement significative quant à la position ou à la forme des pics correspondants.

14. Pince (15) selon la revendication 13, **caractérisée en ce que** les extrémités distales (1511 ou 1521) comportent une lame (15111 ou 15211), et **en ce que** la première branche (151) comporte un point pivotal (1513) de telle manière que lors le serrage des branches (151, 152) la deuxième branche (152) est apte à basculer sur le point pivotal (1513), les extrémités (1511, 1521) sont aptes à se mouvoir l'une vers l'autre et les lames (15111, 15211) sont aptes à glisser en ciseau l'une contre l'autre pendant ce mouvement convergent.

15. Pince selon l'une des revendications 13 ou 14, où l'alliage d'acier est un acier ferritique, martensitique ou mixte ferritique/martensitique, plus préférablement un acier présentant la composition Cr 12,50-14,50 % en poids, C 0,42-0,50 % en poids, Si max. 1,00 % en poids, Mn max. 1,00 % en poids, P max. 0,045 % en poids, S max. 0,030 % en poids, le reste étant essentiellement du fer et des impuretés inévitables.

16. Implant de soutien pour une articulation, comprenant une extrémité distale et une extrémité proximale d'os, où lesdites extrémités d'os sont reliées l'une à l'autre par articulation, et où l'implant de soutien comprend un ruban métallique selon l'une des revendications 1 à 3 ou consiste de celui-ci.

17. Prothèse articulaire pour le remplacement complet d'une articulation, comprenant un ruban métallique selon l'une des revendications 1 à 3 ou consistant d' un ruban métallique selon l'une des revendications 1 à 3.

18. Prothèse articulaire selon la revendication 17 pour le remplacement complet d'une articulation de la mâchoire, le ruban métallique présentant une zone ayant la forme d'une partie de ressort à lame (192) où les cristallites sont orientés avec une anisotropie relativement élevée ou qui est obtenable par le procédé de laminage selon une des revendications 4 à 10, et cinq zones où les cristallites sont préférentiellement orientés avec une anisotropie comparativement moins élevée, ayant la forme d'une partie de mâchoire supérieure (191), d'une partie de branche ascendante (193), d'un partie d'appui (194), d'une partie de branche descendante (195) et d'une partie de mâchoire inférieure (196) ; de telle manière que la partie de ressort à lame (192) est adjacente à la partie de mâchoire supérieure (191), la partie de branche ascendante (193) est adjacente à la partie de ressort à lame (192), la partie d'appui (194) est adjacente à la partie de branche ascendante (193), la partie de branche descendante (195) est adjacente à la partie d'appui (194) et la partie de mâchoire inférieure (196) est adjacente à la partie de branche descendante (195) ; et de telle manière que la partie de ressort à lame (192) et la partie de branche ascendante (193) forment une première boucle, dont l'ouverture pointe vers la partie de mâchoire supérieure (191), et la partie de branche ascendante (193), la partie d'appui (194) et la partie de branche descendante (195) forment une deuxième boucle dont l'ouverture pointe vers la direction opposée ; la partie d'appui (194) et la partie de mâchoire supérieure (191) peuvent se toucher de telle manière que la partie d'appui (194) peut se dérouler sur la partie de mâchoire supérieure (191) et glisser le long de celle-ci, de manière que la prothèse présente les possibilités de mouvement d'une articulation de mâchoire en raison de la partie de ressort à lame (192) et du point de contact entre la partie de mâchoire supérieure (191) et la partie d'appui (194).

# Fig. 1

# Fig. 2

## Fig. 3

1311  131  13  1312  133

1321  132  1322

## Fig. 4

14  1412  143

141

1411

1421  64  142

1422

## Fig. 5

## Fig. 6

# Fig. 7

# Fig. 8

# Fig. 9

**Fig. 10**

**Fig. 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1275472 A
- WO 0113756 A
- US 5001918 A
- US 1898061 A
- US 3457759 A
- DE 1527680 A

**In der Beschreibung aufgeführte Nicht-Patentliteratur**

- **L. Spiess ; R. Schwarzer ; H. Behnken ; G. Teichert.** Moderne Röntgenbeugung. B.G. Teubner Verlag, Oktober 2005
- *J. Appli. Cryst.,* 1970, vol. 3, 313ff